# EUROPEAN PATENT SPECIFICATION

(11) **EP 1 781 813 B1**
(45) Date of publication and mention of the grant of the patent: **27.01.2010**
(21) Application number: 05762161.7
(22) Date of filing: 17.06.2005
(51) Int. Cl.: C12Q 1/68

(54) **COMPOSITIONS AND METHODS FOR PREVENTING CARRY-OVER CONTAMINATION IN NUCLEIC ACID AMPLIFICATION REACTIONS**
ZUSAMMENSETZUNGEN UND VERFAHREN ZUR VERHINDERUNG DES MITSCHLEPPENS EINER KONTAMINATION IN NUKLEINSÄURE-AMPLIFIKATIONSREAKTIONEN
COMPOSITIONS ET METHODES POUR EVITER UNE CONTAMINATION PAR RECIRCULATION DANS DES REACTIONS D'AMPLIFICATION D'ACIDE NUCLEIQUE

(30) Priority: 17.06.2004 US 580638 P; 17.06.2004 US 580529 P; 17.06.2004 US 580644 P
(43) Date of publication of application: 09.05.2007
(73) Proprietor: Epigenomics AG, 10178 Berlin (DE)
(72) Inventor: TETZNER, Reimo, c/o Epigenomics AG, 10178 Berlin (DE); BERLIN, Kurt, c/o Epigenomics AG, 10178 Berlin (DE); DISTLER, Jürgen, c/o Epigenomics AG, 10178 Berlin (DE)
(74) Representative: Krauss, Jan
(86) International application number: PCT/US2005/021525
(87) International publication number: WO 2006/009870

(56) References cited:
- WO-A-02/18649
- WO-A-92/01814
- WO-A-99/07887
- WO-A-03/025215
- WO-A-03/070986
- WO-A-2005/068648
- PERREN AUREL ET AL: "Clonal analysis of sporadic pancreatic endocrine tumours" JOURNAL OF PATHOLOGY, vol. 186, no. 4, December 1998 (1998-12), pages 363-371, XP009047128 ISSN: 0022-3417
- LONGO M C ET AL: "USE OF URACIL DNA GLYCOSYLASE TO CONTROL CARRY-OVER CONTAMINATION IN POLYMERASE CHAIN REACTIONS" GENE, ELSEVIER BIOMEDICAL PRESS. AMSTERDAM, NL, vol. 93, no. 1, January 1990 (1990-01), pages 125-128, XP000371626 ISSN: 0378-1119 cited in the application
- RITTER M ET AL: "Detection of DNA methylation in the calcitonin gene in human leukemias using differential polymerase chain reaction" LEUKEMIA (BASINGSTOKE), vol. 9, no. 5, 1995, pages 915-921, XP009004895 ISSN: 0887-6924

### SEQUENCE LISTING

<110> Epigenomics AG
   Tetzner, Reimo
   Berlin, Kurt
   Distler, Juergen
<120> COMPOSITIONS AND METHODS FOR PREVENTING CARRY-OVER CONTAMINATION IN NUCLEIC ACID AMPLIFICATION REACTIONS
<130> 47675-111
<150> US 60/580,638 <151> 2004-06-17
<150> US 60/580,644 <151> 2004-06-17
<150> US 60/580,529 <151> 2004-06-17
<160> 13
<170> PatentIn version 3.3
<210> 1
   <211> 1826
   <212> DNA
   <213> Homo sapiens
<400> 1
   ctgcagaaac tgcctaggtc ggcccatggc cacggggcgc caatttttca aggaaaagtc     60
   aatgctaata atggtggcaa tcacgggaaa tccattctga ggccagatct gacttgtcag    120
   gattaatcat catttccact taacttcgaa ctgacctggg taaaaacgtg agcgcgaggg    180
   gaccaggctg cacctctgac ctggctcccc tctgcaaaaa tcgcgaagtg ggtgcccgag    240
   gtggggcggg ggttggggga gacctccccg ggagtcccca cccagcctgc tctgcacatc    300
   ttagtccctc atccgcttgc gctgtgcaaa tctgtcttct gtcatttgta tcgcaagaca    360
   tcaaaatccc caaccaaatg caaatactga gacctcataa tctgagacaa agcttcacgg    420
   tatccagaaa gcccccagca ggtgtgcagt gcagagccag ccccccagcg gtcttccgca    480
   gaatcctatc agtttccccc tttcgtgctg tgtgcatcga gcaggaaggg gcttggcagg    540
   ttttacctgc cctctttcct ttctgaaaag tctgggcctc ctcaccccga aaggagtcaa    600
   ctccttgcag ttccccagtt gcgaaaagag gaggaagttg gctgggccgg gggccgcggg    660
   gggcaccctc cgcagatggc gggacccccc tgccggccat ggcaaaaacg aggcttgtct    720
   ctcccaccgc ccccaacctt agtccttggc acattgttga aagtaattga ataaaatcgg    780
   aaattcgaga aggcgttcgt tcggattggt gagattttga ggggagaaag aagcggggac    840
   ttcgccggca ccagcggcgc cccctcctcg gccaccgtta acccccattc cagagggcac    900
   tgccccgcca cccagcctag gtccccctgc gagagcctcg cgggcccgcg cagcctccgc    960
   gactcgaaca gatcttcagt ccttggagga atgcctgttt ctctaacaat aaaaaattaa   1020
   agaagcgctc ataaatgcca agtcctctcg cactatgcgg agtacagagg acaacgacca   1080
   cagccatccc tgaaccccgc ccacggcaca gcgccggagc cggggtctgg ggcgccgctt   1140
   cctggggggt cccgactctc agccgccccc gctccacccg ggccgccaag gggctggggg   1200
   aggcggcgct cggggtaacc gggggagact cagggcgctg ggggcacttg gggaactcat   1260
   gggggctcaa aggaactagg agatcggggc ctcgaagggg acttgggggg ttcggggctt   1320
   tcgggggcgg tcgggggttc gcggacccgg gaagctctga ggacccagag gccgggcgcg   1380
   ctccgcccgc ggcgccgccc cctccgtaac tttcccagtc tccgagggaa gaggcggggt   1440
   gtggggtgcg gttaaaaggc gccacggcgg gagacaggtg ttgcggcccc gcagcgcccg   1500
   cgcgctcctc tccccgactc ggagcccctc ggcggcgccc ggcccaggac ccgcctagga   1560
   gcgcaggagc cccagcgcag agaccccaac gccgagaccc ccgccccggc cccgccgcgc   1620
   ttcctcccga cgcaggtgag cccgccggcc ccggactgcc cggccaggaa cctggcgcgg   1680
   ggagggaccg cgagacccag agcggttgcc cggccgcgtg ggtctcgggg aaccgggggg   1740
   ctggaccaac acacgtcctt gggccggggg gcgggggccg ccttctggag cgggcgtttc   1800
   tgcggccgag ctccggagct ggaatg                                         1826
<210> 2
   <211> 43
   <212> DNA
   <213> Artificial sequence
<220>
   <223> amplification primer
<400> 2
   cctctaaaat aaaaattaac aataaccaaa aaaaaaacac cac 43
<210> 3
   <211> 21
   <212> DNA
   <213> Artificial sequence
<220>
   <223> Detection probe 1
<400> 3
   ggagaaagaa gcggggattt c 21
<210> 4
   <211> 22
   <212> DNA
   <213> Artificial sequence
<220>
   <223> Detection probe 2
<400> 4
   cggtattagc ggcgtttttt tt 22
<210> 5
   <211> 30
   <212> DNA
   <213> Artificial sequence
<220>
   <223> Primer 6211.4F1
<400> 5
   ggtatattgt tgaaagtaat tgaataaaat 30
<210> 6
   <211> 29
   <212> DNA
   <213> Artificial sequence
<220>
   <223> Primer 6211.4R1
<400> 6
   aaacaatacc ctctaaaata aaaattaac 29
<210> 7
   <211> 31
   <212> DNA
   <213> Artificial sequence
<220>
   <223> primer 6211.4F-Alu
<400> 7
   ggtatattgt tgaaagctaa ttgaataaaa t 31
<210> 8
   <211> 30
   <212> DNA
   <213> Artificial sequence
<220>
   <223> Primer 6211.4R-Bfa1
<400> 8
   aaacaatacc ctctagaaat aaaaattaac 30
<210> 9
   <211> 30
   <212> DNA
   <213> Artificial sequence
<220>
   <223> Primer 6211.4F-Hpy
<400> 9
   ggtatattgt tgaaagtaat tgcataaaat 30
<210> 10
   <211> 29
   <212> DNA
   <213> Artificial sequence
<220>
   <223> Primer 6211.4F-Bfa-2
<400> 10
   aaacaatacc ctctagaata aaaattaac 29
<210> 11
   <211> 30
   <212> DNA
   <213> Artificial sequence
<220>
   <223> Primer 6211.4F1
<400> 11
   ggtatattgt tgaaagtaat tgaataaaat 30
<210> 12
   <211> 29
   <212> DNA
   <213> Artificial sequence
<220>
   <223> Primer 6211.4R1
<400> 12
   aaacaatacc ctctaaaata aaaattaac 29
<210> 13
   <211> 158
   <212> DNA
   <213> Artificial sequence
<220>
   <223> amplificate of restriction site location
<400> 13
   ggtatattgt tgaaagtaat tgaataaaat cggaaattcg agaaggcgtt cgttcggatt     60
   ggtgagattt tgaggggaga aagaagcggg gatttcgtcg gtattagcgg cgtttttttt    120
   tcggttatcg ttaattttta ttttagaggg tattgttt                            158

## Description

### FIELD OF THE INVENTION

The invention generally relates generally to nucleic acid amplification reactions and more particularly to novel compositions and methods for preventing carry-over contamination within nucleic acid amplification reactions.

### BACKGROUND

In recent decades, molecular biology studies have focused primarily on genes, the transcription of those genes into RNA, and the translation of the RNA into protein. There has been a more limited analysis of the regulatory mechanisms associated with gene control. Gene regulation, for example, at what stage of development of the individual a gene is activated or inhibited, and the tissue specific nature of this regulation is less well understood. However, such regulation can be with the extent and nature of methylation of the gene or genome. Specific cell types can be correlated with specific methylation patterns, as has been shown for a number of cases (Adorjan et al. (2002) Tumour class prediction and discovery by microarray-based DNA methylation analysis. Nucleic Acids Res. 30 (5) e21).

In higher order eukaryotes, DNA is methylated nearly exclusively at cytosines located 5' to guanine in the CpG dinucleotide. This modification has important regulatory effects on gene expression, especially when involving CpG rich areas, known as CpG islands located in the promoter regions of many genes. While almost all gene-associated islands are protected from methylation on autosomal chromosomes, extensive methylation of CpG islands has been associated with transcriptional inactivation of selected imprinted genes and genes on the inactive X-chromosome of females.

Cytosine modification, in form of methylation, contains significant information. The identification of 5-methylcytosine in a DNA sequence, as opposed to unmethylated cytosine; that is, the methylatio status, is of great importance and warrants further study. However, because 5-methylcytosine behaves like cytosine in terms of hybridization preference (a property relied on for sequence analysis), its positions/status can not be identified by a normal sequencing reaction. Furthermore, in any amplification, such as a PCR amplification, this relevant epigenetic information, methylated cytosine or unmethylated cytosine, will be lost completely.

Several methods are known in the art that relate to this problem. Usually genomic DNA is treated with a chemical or enzyme leading to a conversion of the cytosine bases, which consequently allows subsequent base differentiatation. The most common methods are: a) the use of methylation sensitive restriction enzymes capable of differentiating between methylated and unmethylated DNA; and b) the treatment with a bisulfite reagent. The use of said enzymes is limited due to the selectivity of the restriction enzyme towards a specific recognition sequence.

Therefore, the specific reaction of bisulfite with cytosine, which, upon subsequent alkaline hydrolysis is converted to uracil (whereas 5-methylcytosine remains unmodified under these conditions) (Shapiro et al. (1970) Nature 227: 1047) is currently the most frequently used method for analyzing DNA for 5-methylcytosine. Uracil corresponds to thymine in its base pairing behaviour; that is, it hybridizes to adenine; whereas 5-methylcytosine does not change its chemical properties under this treatment, and therefore still has the base pairing behavior of a cytosine (hybridizing with guanine). Consequently, the original DNA is converted in such a manner that 5-methylcytosine, which originally could not be distinguished from cytosine by its hybridization behavior, can now be detected as the only remaining cytosine using standard molecular biological techniques, for example, amplification and hybridization or sequencing. All of these techniques are based on base pairing, which can now thereby be more fully exploited. Comparing the sequences of the DNA with and without bisulfite treatment allows an easy identification of those cytosines that have been unmethylated. An overview of further known methods for detecting 5-methylcytosine may be gathered from the following review article: Rein T, DePamphilis ML, Zorbas H (1998), Nucleic Acids Res., 26: 2255.

A very sensitive method that encloses the DNA to be analyzed in an agarose matrix, thus preventing diffusion and renaturation of the DNA (bisulfite reacts with single-stranded DNA only), and which replaces all precipitation and purification steps with fast dialysis is described by Olek et al. (Olek A, Oswald J, Walter J. (1996) A modified and improved method for bisulfite based cytosine methylation analysis. Nucleic Acids Res. 24: 5064-6). Using this method, it is possible to analyze individual cells, which illustrates the potential of the method.

To date, barring few exceptions (e.g., Zeschnigk M, Lich C, Buiting K, Doerfler W, Horsthemke B. (1997) A single-tube PCR test for the diagnosis of Angelman and Prader-Willi syndrome based on allelic methylation differences at the SNRPN locus. Eur J Hum Genet. 5: 94-8) the bisulfite technique is only used in research. Always, however, short, specific fragments of a known gene are amplified subsequent to a bisulfite treatment and either completely sequenced (Olek A, Walter J. (1997). The pre-implantation ontogeny of the H19 methylation imprint. Nat Genet. 3: 275-6) or individual cytosine positions are detected by a primer extension reaction (Gonzalgo ML and Jones PA. (1997). Rapid quantitation of methylation differences at specific sites using methylation-sensitive single nucleotide primer extension (Ms-SNuPE). Nucleic Acids Res. 25 :2529-31, WO 95/00669) or by enzymatic digestion (Xiong Z, Laird PW. (1997) COBRA: a sensitive and quantitative DNA methylation assay. Nucleic Acids Res. 25: 2535-4).

Another technique to detect hypermethylation is the so-called methylation specific PCR (MSP) (Herman JG, Graff JR, Myohanen S, Nelkin BD and Baylin SB. (1996), Methylation-specific PCR: a novel PCR assay for methylation status of CpG islands. Proc Natl Acad Sci U S A. 93: 9821-6). The technique is based on the use of primers that differentiate between a methylated and a non-methylated sequence if applied after bisulfite treatment of said DNA sequence. The primer either contains a guanine at the position corresponding to the cytosine in which case it will after bisulfite treatment only bind if the position was methylated. Alternatively, the primer contains an adenine at the corresponding cytosine position and therefore only binds to said DNA sequence after bisulfite treatment if the cytosine was unmethylated and has hence been altered by the bisulfite treatment so that it hybridizes to adenine. With the use of these primers, amplicons can be produced specifically depending on the methylation status of a certain cytosine and will as such indicate its methylation state.

Another new technique is the detection of methylation via Taqman PCR, also known as MethylLight^{™} (WO 00/70090). With this technique it became feasible to determine the methylation state of single or of several positions directly during PCR, without having to analyze the PCR products in an additional step. In addition, detection by hybridization has also been described (Olek et al., WO 99/28498).

Further publications dealing with the use of the bisulfite technique for methylation detection in individual genes are: Grigg G, Clark S. (1994) Sequencing 5-methylcytosine residues in genomic DNA. Bioessays 16: 431-6; Zeschnigk M, Schmitz B, Dittrich B, Buiting K, Horsthemke B, Doerfler W. (1997) Imprinted segments in the human genome: different DNA methylation patterns in the Prader-Willi/Angelman syndrome region as determined by the genomic sequencing method. Hum Mol Genet. 6, 387-395; Feil R, Charlton J, Bird AP, Walter J, Reik W (1994) Methylation analysis on individual chromosomes: improved protocol for bisulphite genomic sequencing. Nucleic Acids Res. 22, 695-696; Martin V, Ribieras S, Song-Wang X, Rio MC, Dante R (1995) Genomic sequencing indicates a correlation between DNA hypomethylation in the 5' region of the pS2 gene and its expression in human breast cancer cell lines. Gene 157,261-264; WO 97/46705, WO 95/15373 and WO 97/45560.

Base excision repair occurs *in vivo* to repair DNA base damage involving relatively minor disturbances in the helical DNA structure, such as deaminated, oxidized, alkylated or absent bases. Numerous DNA glycoslylases are known in the art, and function *in vivo* during base excision repair to release damaged or modified bases by cleavage of the glycosidic bond that links such bases to the sugar phosphate backbone of DNA (Memisoglu, Samson, Mutation Res. (2000), 451:39-51). All DNA glycosylases cleave glycosidic bonds but differ in their base substrate specificity and in their reaction mechanisms.

One widely recognized application of such glycosylases is decontamination in PCR applications. In any such PCR amplification, 2 to the 30 (2³⁰) or more copies of a single template are generated. This very large amount of DNA produced helps in the subsequent analysis, like in DNA sequencing according to the Sanger method, but it can also become a problem when this amount of DNA is handled in an analytical laboratory. Even very small reaction volumes, when inadvertently not kept in a closed vial, can lead to contamination of the whole work environment with a huge number of DNA copies. These DNA copies may be templates for a subsequent amplification experiment performed, and the DNA analysed subsequently may not be the actual sample DNA, but contaminating DNA from a previous experiment. This may also lead to positive negative controls that should not contain any DNA and therefore no amplification should be observed. In practice, this problem can be so persistent that whole laboratories may move to a new location, because contamination of the work environment makes it impossible to still carry out meaningful PCR based experiments. In a clinical laboratory, however, the concern is also that contaminating DNA may cause false results when performing molecular diagnostics. This would mean that actually contaminating DNA that stems from a previous patient is analysed, instead of the actual sample to be investigated.

Therefore, measures have been implemented to avoid contamination. This involves, for example, a PCR amplification and detection in one tube in a real time PCR experiment. In this case, it is not required that a PCR tube be opened. After use, the tube will be kept closed and discarded and therefore the danger of contamination leading to false results is greatly reduced.

Additionally, molecular means exist that reduce the risk of contamination. In a polymerase chain reaction, the enzyme uracil-n-glycosylase reduces the potential for false positive reactions due to amplicon carryover (see e.g. Thornton CG, Hartley JL, Rashtchian A (1992). Utilizing uracil DNA glycosylase to control carryover contamination in PCR: characterization of residual UDG activity following thermal cycling. Biotechniques. 13(2):180-4).

In any amplification dUTP is used instead of dTTP and the resulting amplicon can be distinguished from its template and any future sample DNA by uracil being present instead of thymine. Prior to any subsequent amplification, uracil n-glycosylase (UNG) is used to cleave these bases from any contaminating DNA, and therefore only the legitimate template remains intact and can be amplified. This method is considered the closest related art and is widely used in DNA based diagnostics.

Polymerase chain reactions (PCRs) synthesize abundant amplification products. Contamination of new PCRs with trace amounts of these products, called carry-over contamination, yields false positive results. Carry-over contamination from some previous PCR can be a significant problem, due both to the abundance of PCR products, and to the ideal structure of the contaminant material for re-amplification (Longo MC, Berninger MS, Hartley JL (1990). Use of uracil DNA glycosylase to control carry-over contamination in polymerase chain reactions. Gene. 1990 Sep 1;93(1):125-8.). Carry-over contamination can be controlled by the following two steps: (i) incorporating dUTP in all PCR products (by substituting dUTP for dTTP, or by incorporating uracil during synthesis of the oligodeoxyribonucleotide primers; and (ii) treating all subsequent fully preassembled starting reactions with uracil DNA glycosylase (UDG), followed by thermal inactivation of UDG. UDG cleaves the uracil base from the phosphodiester backbone of uracil-containing DNA, but has no effect on natural (i.e., thymine-containing) DNA. The resulting apyrimidinic sites block replication by DNA polymerases, and are very labile to acid/base hydrolysis. Because UDG does not react with dUTP, and is also inactivated by heat denaturation prior to the actual PCR, carry-over contamination of PCRs can be controlled effectively if the contaminants contain uracils in place of thymines (*Id*).

Another method for carry over protection in PCR has been described by Walder et al ( Walder RY, Hayes JR, Walder JA Use of PCR primers containing a 3-terminal ribose residue to prevent cross-contamination of amplified sequences. Nucleic Acids Res 1993 Sep 11;21(18):4339-43.).

Walder teaches that carry over protection can be achieved, at least to some extent (and not very reproducibly), by using primers consisting of a 3'-end that is characterized as a ribocytidine. After primer extension, the amplification product is cleaved specifically at the site of this ribonucleotide by an enzyme known as RNase A. In this manner, the potentially contaminating amplificates are shortened at their ends and cannot serve a templates for said primers in the following amplification procedure. However, a substantial disadvantage inherent to this method is the instability of the primer molecules, containing a ribonucleotide at the 3'-end.

### SUMMARY OF THE INVENTION

Aspects of the present invention provide surprisingly effective alternatives to the carry over protection system known as UNG system, and other art-recognized methods as described above under "BACKGROUND."

Particular aspects provide methods for the specific amplification of template DNA in the presence of potentially contaminating PCR products from previous amplification experiments, but wherein the contaminating prior reaction amplificates (carry-over contaminants) are rendered non-amplifiable. In a first step, a pair of primer oligonucleotides and a sample DNA template is provided, wherein at least one of the primers is complementary (e.g., is fully or completely complementary) to any contaminating DNA, but forms mismatch(es) (in the corresponding region) with the sample DNA template. For example, at least one of the primers is fully complementary to any contaminating prior reaction amplificate nucleic acid, but contains a mismatch with the correspond sequence of the sample template nucleic acid. Contaminating DNA is specifically enzymatically degraded, and only the sample template DNA is thereafter amplifiable in a subsequent sample template amplification step. The method is surprisingly effective, and has substantial utility for the decontamination of amplification template DNA samples, or more specifically for preclusion of amplification of 'carry-over products,' and has particularly substantial utility in the in context of DNA methylation analysis because, unlike prior art methods, the present inventive methods are compatible with methods using bisulfite-treated DNA as amplification templates.

Additional aspects provide a method for the specific amplification of single-stranded sample template DNA in the presence of potentially contaminating double-stranded PCR products from previous amplification experiments (carry-over contamination). The method comprises adding a pre-incubation step prior to the intended PCR amplification. During this pre-incubation, a specific restriction digest is performed by a preferably thermolabile restriction enzyme, selectively cleaving the double-stranded prior reaction PCR product, but not the single-stranded sample template nucleic acid. Any contaminating DNA is subsequently degraded or at least fragmented enzymatically and only the sample DNA is amplified in the next step. The method is useful for the decontamination of single-stranded DNA samples or rather the inhibition of amplification of 'carry over products,' in particular in the context of DNA methylation analysis.

Further aspects provide methods comprising use of a template-dependent thermostable DNA polymerase enzyme suitable for incorporating ribonucleotides as well as deoxy-nucleotides when copying a template (*e.g.,* amplifying), consisting of deoxy-nucleotides (DNA) or of deoxynucleotides and ribonucleotides (chimeric nucleic acids), in the presence of at least one ribonucleoside-triphosphate (*e.g.,* adenosin-triphosphate (ATP) or uridine-triphosphate (UTP)). Such polymerases are referred to herein as "ribonucleotide tolerant polymerases" in the context of this invention. Such chimeric PCR products are sensitive to enzymes (*e.g.,* RNases and other cleaving enzymes) that recognize ribonucleotides, and can thereby be destroyed, digested or fragmented. Thus, any contaminating prior amplificates are therefore eliminated by treatment with such enzymes prior to sample template amplification, and false-positive results are thus avoided. The inventive carry-over protection treatment can be done in a "one tube reaction" with the final PCR reaction, and there is no need to open the tube between said enzymatic digest and the PCR start. Preferably, the RNase or fractionating enzyme is thermolabile, and is specific for double-stranded nucleic acids comprising ribonucleotides and deoxynucleotides, or specific for double-stranded and single-stranded chimeric nucleic acids comprising ribonucleotides and deoxynucleotides. Preferably, the enzyme hydrolyses the phosphodiester bond of a ribonucleotide and the nucleotide attached to this residue. Exemplary enzymes having utility for this purpose are RNase H, which is known to specifically cleave DNA/RNA hybrids, and RNase III, which is known to specifically cleave double-stranded RNA in an endonucleolytic way. RNase If is described to specifically cleave single-stranded RNA in an exonucleolytic manner.

After cleavage, fragmentation, digestion, etc. of any contaminating prior chimeric amplificate,. the enzyme (*e.g.,* thermolabile) will be inactivated (*e.g.,* heat) in, for example, the initial denaturation step of a PCR reaction. The inventive system is surprisingly effective,a nd eliminates PCR product contamination in PCR from genomic DNA, and is particularly well suited for use in methylation analyses with bisulfite-converted DNA.

### BRIEF DESCRIPTION OF THE DRAWINGS

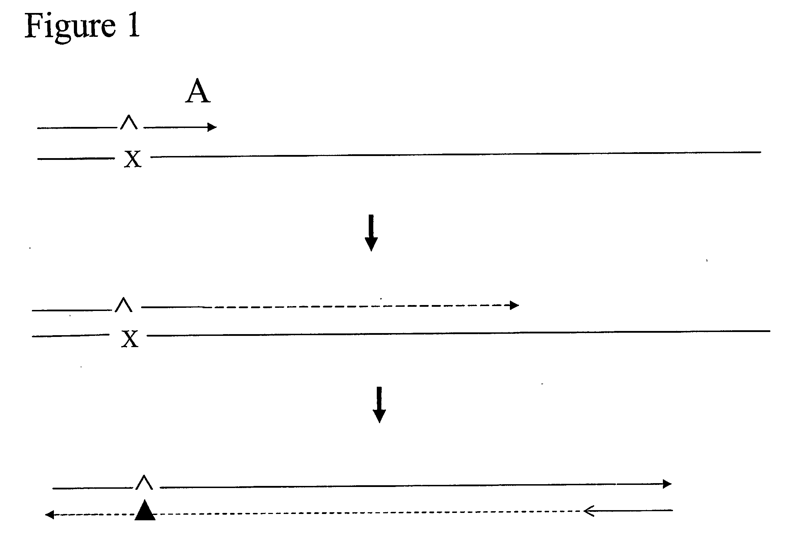

Figure 1 shows, according to particular aspects of the present invention, a primer A with a mismatch to a template elongation of the primer copying the first strand with a second primer and thereby duplicating the mismatched nucleotide that was incorporated by primer A. At the top of the figure, primer A binds to a template nucleic acid, mispriming at a single nucleotide X in the sample DNA. Mismatched positions do not prevent the primer from binding to the sample DNA. At the middle of the figure, primer A is extended, reproducing the complementary strand to the single-stranded template nucleic acid from the sample DNA. At the bottom of the figure, a second primer B binds to the generated complementary strand, and a polymerase extends it thereby producing the complementary strand to the first copied strand; that is, a strand identical to the template nucleic acid, but differing in exactly one position: at position X a new nucleotide is introduced instead of the one in the sample DNA. The absence, presence or properties of said amplificate can now be analysed.

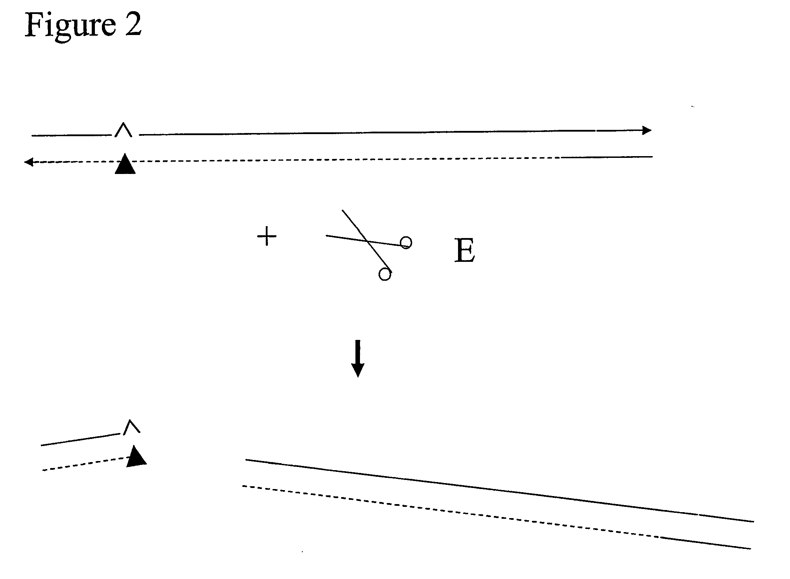

Figure 2 shows a restriction digest that results in excision of the "wrongly incorporated" (mismatched) nucleotides. The figure shows introduction of the new nucleotide introducing a restriction site, which is unique in the amplificate and specific for the amplified nucleic acids. Before starting the next amplification, the PCR reaction mixture is treated with a restriction enzyme (E) digest The perfectly matched position in the amplificate is recognized as the introduced restriction site by restriction enzyme E, and the double stranded amplificate is cleaved at this position. The amplificate end is shortened sufficiently to hinder the hybridisation to any new primer A.

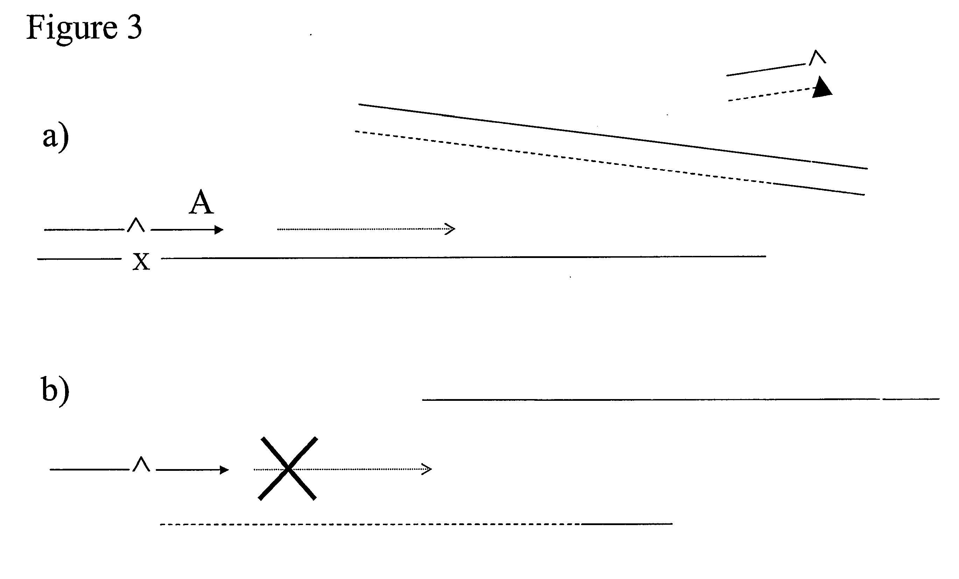

Figure 3 shows (upper row (a)) how, in presence of a digested prior reaction amplification product (digested contaminants), the new template (sample template DNA)is amplified again introducing a mismatch, and (lower row (b)) how a digested (single-stranded) contamination product cannot serve as a sufficient template for the primer anymore (too short). Briefly, a new sample DNA can be amplified, in presence of the contaminating remains of the previous sample amplificate, which was treated with the restriction enzyme beforehand. The primer A is only binding to the new single-stranded template nucleic acid from the new sample, mispriming at a single nucleotide (X) in the sample DNA. Mismatched positions do not prevent the primer from binding to the sample DNA. The digested remains of the previous sample amplificate (also single stranded after denaturation) do not work as a template for primer A because the primer cannot bind sufficiently. The cleaved contaminating DNA is too short to significantly bind to primer A. The overlap of the complementary sequences is too short. Therefore the cleaved contaminating DNA is not copied by a polymerase, whereas the sample DNA is copied by polymerase D with A as the primer.

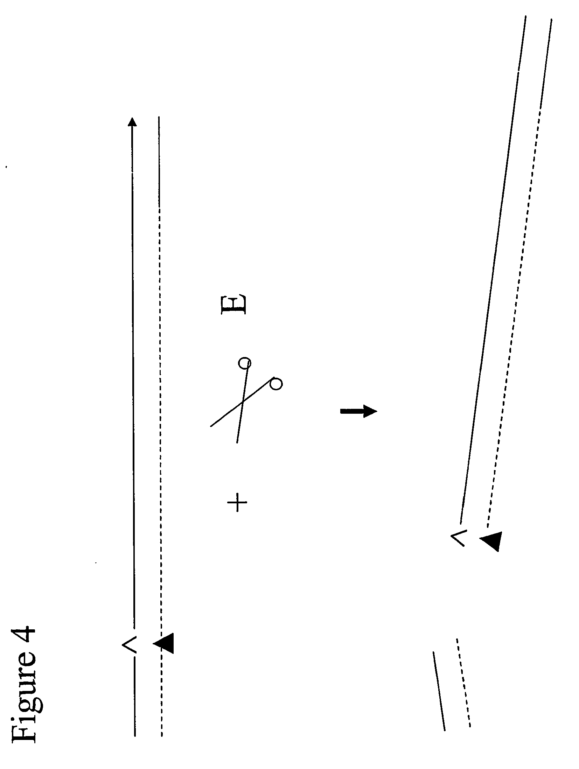

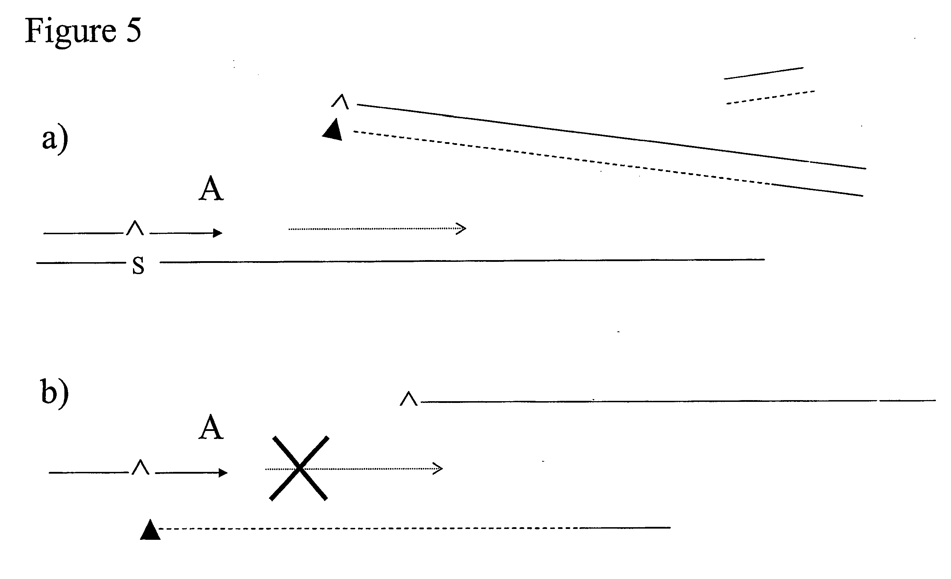

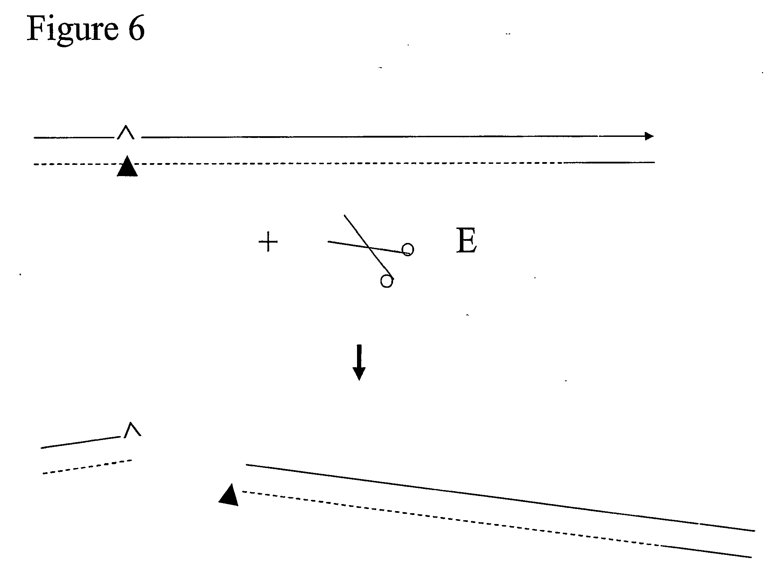

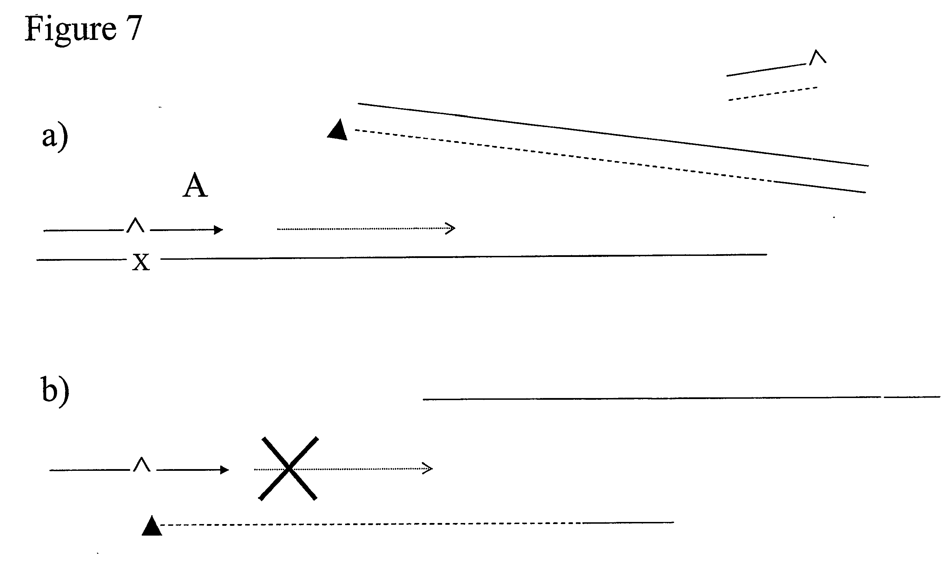

Figures 4 and 5, and Figures 6 and 7 respectively, represent the same scenarios as described in Figure 3, but for different cutting sites. Wherein in Figure 2 the enzyme could represent a blunt-end cutter, cleaving the amplificate such that the introduced nucleotide is cut off from the remaining amplificate, Figure 4 illustrates an embodiment where an enzyme cleaves the amplificate such that the introduced nucleotide is left with the remaining amplificate, which does not detract from the effectiveness of the method. In Gigure 4 the enzyme is shown producing an overhang resulting in the situation that the nucleotide introduced as mismatch remains with one strand of the amplified fragment, and the complement nucleotide remains with one strand of the short cleaved nucleic acid.

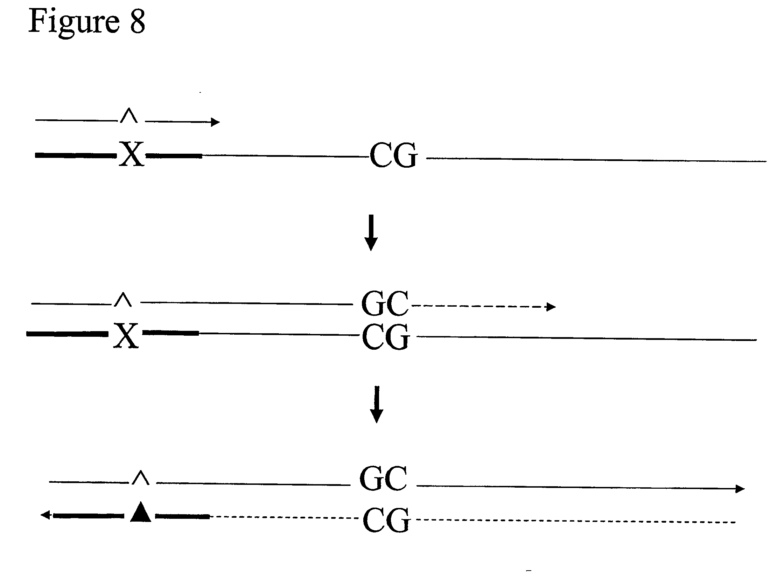

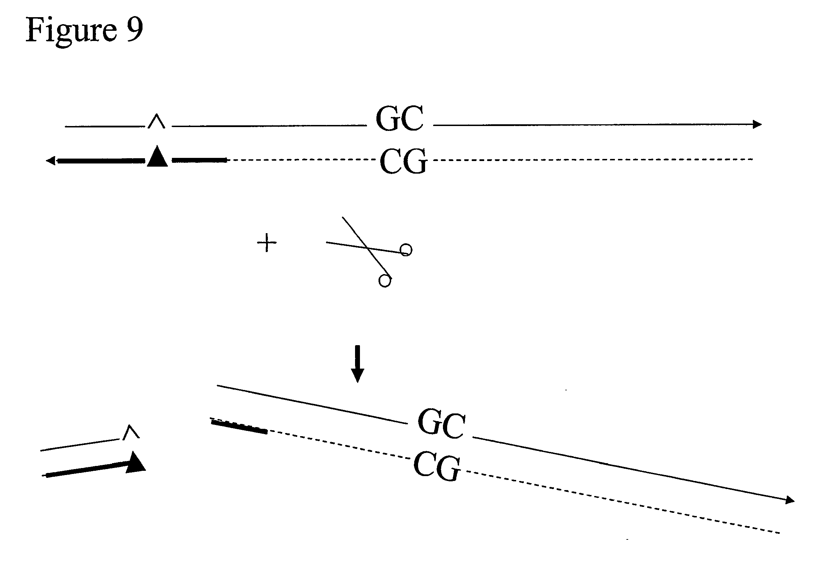

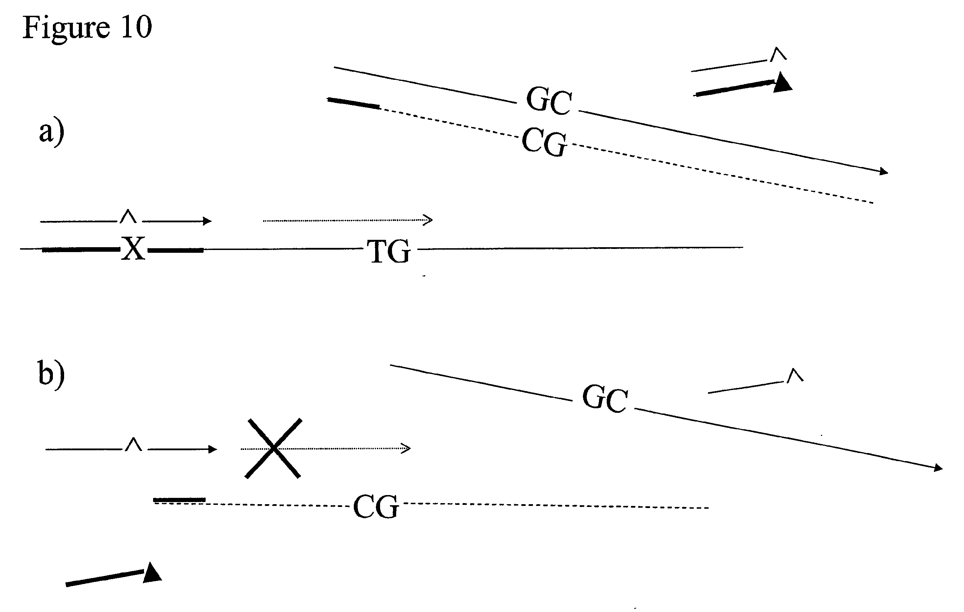

Figures 8, 9 and 10 illustrate scenarios analogous to those in Figures 1, 2 and 3, but specific for a methylation detection scenarios, wherein a bisulfite treated template is amplified and a CG position is detected in the first sample whereas a TG position is detected in the second sample, and false positives, caused by the contaminating DNA from a previous sample are avoided; a substantial improvement over the are, because diagnostically, a TG might indicate "healthy," whereas CG might indicate "cancer."

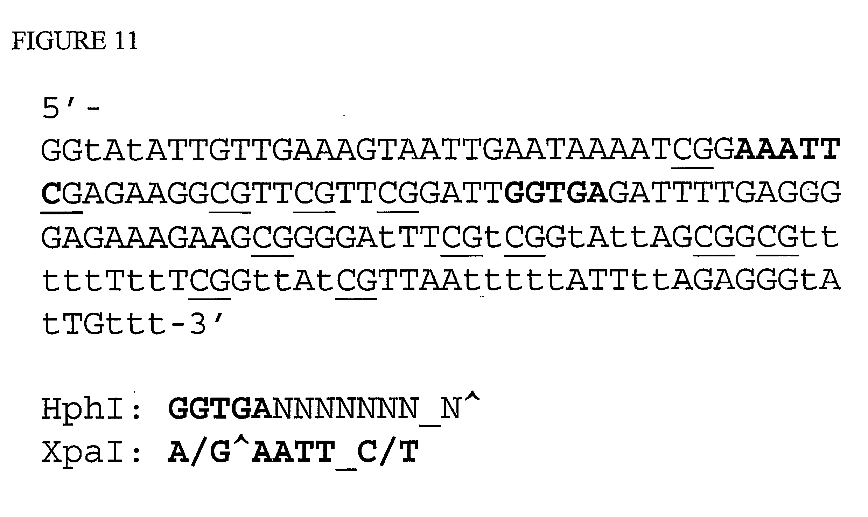

Figure 11 shows the location of restriction sites withing an amplificate according to Example II herein below.

### DETAILED DESCRIPTION OF THE INVENTION

### Definitions

The term "primer" as used herein refers to an oligonucleotide primer, whether natural or synthetic, which is capable of acting as a point of initiation of nucleic acid synthesis when placed under conditions in which primer extension (not limited in number of extended bases) is initiated. A primer is preferably a single-stranded oligodeoxyribonucleotide. The appropriate length of a primer, as appreciated in the relevant art, depends on the intended use of the primer but typically ranges from about 15 to about 35 nucleotides. Short primer molecules generally require cooler temperatures to form sufficiently stable hybrid complexes with the template. A primer need not reflect the exact sequence of the template but must be sufficiently complementary to hybridize with a template for primer elongation to occur. A primer can be labeled, if desired, by incorporating a label that is detectable by, for example, spectroscopic, photochemical, biochemical, immunochemical, or chemical means. Exemplary labels include, but are not limited to radiolabels (*e.g.,* ³²P), fluorescent dyes, electron-dense reagents, enzymes (as commonly used in ELISAS), biotin, or haptens and proteins for which antisera or monoclonal antibodies are available.

The term "thermostable polymerase," refers to an enzyme which is stable to heat (*e.g.,* heat resistant) and retains sufficient activity to effect subsequent primer extension reactions when subjected to the elevated temperatures for the time necessary to effect denaturation of double-stranded nucleic acids. Heating conditions necessary for nucleic acid denaturation are well known in the art and are exemplified in U.S. Patent Nos. 4,683,202 and 4,683, 195. As used herein, a thermostable polymerase is suitable for use in a temperature cycling reactions such as the polymerase chain reaction ("PCR"). For a thermostable polymerase, enzymatic activity refers to the catalysis of the combination of the nucleotides in the proper manner and order to form primer extension products that are complementary to a template nucleic acid strand.

The term "ribonucleotide tolerant polymerase" refers to template-dependent thermostable DNA polymerase enzyme, characterized as being capable of incorporating ribonucleotides as well as other unconventional ribonucleotides, as well as deoxy-ribonucleotides when copying a template consisting of deoxy-nucleotides only. Such enzymes are described in detail in US patent 5,939,292 to Gelfand et al..

Most common polymeric or oligomeric nucleic acids consist of one type of nucleotide only, ribonucleotides or deoxy-ribonucleotides. "Deoxy-nucleic acids" as used herein refers to DNA, comprised of deoxy-ribonucleotides. "Ribonucleic acids" as used herein refers to RNA, comprised of ribonucleotides. In the context of this application deoxy-ribonucleotides will sometimes be referred to as deoxy-nucleotides.

"Chimeric nucleic acid" as used herein refers to an oligomeric or polymeric nucleic acid which comprises both nucleotides (deoxynucleotides and ribonucleotides) within in one strand.

"Hybrid nucleic acid" or "hybrid DNA" as used herein refers to an oligomeric or polymeric nucleic acid which comprises of two strands, one strand consisting of deoxynucleotides and the other one consisting of ribonucleotides.

As used herein, the term "reactants" as used herein in the context on nucleic acid amplification reactions refers to a reaction mixture that comprises elements necessary for a nucleic acid amplification (*e.g.,* by PCR). Thus, a nucleic acid amplification reaction mixture is suitable for use in a nucleic acid amplification method. Typically, a nucleic acid amplification reaction will comprise a buffer, suitable for polymerization activity, deoxyribonucleoside triphosphates and at least one unconventional nucleotide (*e.g.,* a ribonucleotide), at least one primer suitable for extension on a target by a polymerase enzyme, a polymerase and a target nucleic acid (*e.g.,* amplification template). Either the primer or one of the nucleotides may be labeled with a detectable moiety such as a fluorescent label. In particular embodiments, the reaction is a mixture that comprises three conventional nucleotides and at least one unconventional nucleotide. In preferred embodiments, the polymerase is a ribnucleotide-tolerent thermostable DNA polymerase and the unconventional nucleotide is a ribonucleotide.

*Methylation Assay Procedures.* Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, and a number of PCR based methylation assays, some of them - known as COBRA, MS-SNuPE, MSP, nested MSP, HeavyMethyl^{™} and MethyLight^{™}-are described in more detail now.

"Bisulfite sequence DNA" as used herein refers to refered to analysis of DNA methylation patterns and 5-methylcytosine distribution as analyzed by sequencing of a previously amplified fragment of bisulfite treated genomic DNA, for example, as described by Frommer et al. (Frommer et al. Proc. Natl. Acad. Sci. USA 89:1827-1831, 1992). As the bisulfite treated DNA is amplified before sequencing, primers according to the invention may be used in the amplification step.

"COBRA" analysis, as used herein, refers to the art-recognized quantitative methylation assay useful for determining DNA methylation levels at specific gene loci in small amounts of genomic DNA (*e.g.,* Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992) or as described by Olek et al (Olek A, Oswald J, Walter J. (1996) Nucleic Acids Res. 24: 5064-6). PCR amplification of the bisulfite converted DNA is then performed using methylation unspecific primers followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples. Typical reagents (*e.g.,* as might be found in a typical COBRA-based kit) for COBRA analysis may include, but are not limited to: PCR primers for specific gene (or methylation-altered DNA sequence or CpG island); restriction enzyme and appropriate buffer; gene-hybridization oligo; control hybridization oligo; kinase labeling kit for oligo probe; and radioactive nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components. Additionally, restriction enzyme digestion of PCR products amplified from bisulfite-converted DNA is also used, in the method described by Sadri & Hornsby (Nucl. Acids Res. 24:5058-5059, 1996).

In particular aspects of the invention (see EXAMPLE 1 herein below), at least one primer used to amplify bisulfite-treated template DNA in a COBRA assay or an assay as described by Sadri & Hornsby, comprises at least one mismatch with respect to priming (annealing) to the sample template DNA, but nonetheless primes without mismatch to any prior reaction amplified DNA (carry-over contamination) thereby introducing a restriction site suitable for use in selectively degrading/cleaving the contaminating nucleic acid.

"Ms-SNuPE" (Methylation-sensitive Single Nucleotide Primer Extension) as used herein refers to the art-recognized quantitative method for assessing methylation differences at specific CpG sites based on bisulfite treatment of DNA, followed by single-nucleotide primer extension (*e.g,* Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using *PCR primers* specific for bisulfite-converted DNA, and the resulting product is isolated and used as a template for methylation analysis at the CpG site(s) of interest. Small amounts of DNA can be analyzed (e.g., microdissected pathology sections), and it avoids utilization of restriction enzymes for determining the methylation status at CpG sites. Typical reagents (e.g., as might be found in a typical Ms-SNuPE-based kit) for Ms-SNuPE analysis may include, but are not limited to: PCR primers for specific gene (or methylation-altered DNA sequence or CpG island); optimized PCR buffers and deoxynucleotides; gel extraction kit; positive control primers; Ms-SNuPE primers for specific gene; reaction buffer (for the Ms-SNuPE reaction); and radioactive nucleotides. Additionally, bisulfite conversion reagents may include: DNA denaturation buffer; sulfonation buffer; DNA recovery regents or kit (e.g., precipitation, ultrafiltration, affinity column); desulfonation buffer; and DNA recovery components.

In particular aspects of the invention (see EXAMPLE 1 herein below), at least one of the primers used to amplify the bisulfite-treated template DNA in a first step, to produce a template for the SNuPE primer and its extension in a second step, comprises at least one mismatch when priming to the sample template DNA, but primes without mismatch to any prior reaction amplified DNA (carry-over contamination), thereby introducing a restriction site suitable for use in selectively degrading/cleaving the contaminating nucleic acid.

"MSP" (methylation-specific PCR) as used herein, refers to the art-recognized method allowing for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP primer pairs contain at least one primer which hybridizes to a bisulfite treated CpG dinucleotide. Therefore, the sequence of said primers comprises at least one CpG dinucleotide. MSP primers specific for non-methylated DNA contain a "T' at the 3' position of the C position in the CpG. Preferably, therefore, the base sequence of said primers is required to comprise a sequence having a length of at least 9 nucleotides which hybridizes to the bisulfite converted nucleic acid sequence, wherein the base sequence of said oligomers comprises at least one CpG dinucleotide. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific gene (or methylation-altered DNA sequence or CpG island), optimized PCR buffers and deoxynucleotides, and specific probes.

In particular aspects of the invention (see EXAMPLE 1 herein below), at least one of the methylation specific primers used to amplify a bisulfite-treated template DNA comprises at least one mismatch with respect to priming (annealing) to the sample template DNA, but nonetheless primes without mismatch to any prior reaction amplified DNA (carry-over contamination) thereby introducing a restriction site suitable for use in selectively degrading the contaminating nucleic acid.

"NESTED MSP" as used herein refers to the art-recognised method described by, for example, Belinsky and Palmisano in US application 20040038245. Considering the apparent conflict of requiring high specificity of the MSP primer to sufficiently differentiate between CG and TG position, while at the same time allowing for a 'mismatch' to create a unique restriction site, it is preferable to use an amended version of MSP, known as nested MSP, as described in WO 02/18649 and US patent application 20040038245 by Belinsky and Palmisano. This method is used to detect the presence of gene-specific promoter methylation, and comprises the steps of: expanding the number of copies of the genetic region of interest by using a polymerase chain reaction to amplify a portion of said region where the promoter methylation resides, thereby generating an amplification product; and using an aliquot of the amplification product generated by the first polymerase chain reaction in a second, methylation-specific, polymerase chain reaction to detect the presence of methylation. In other words, a non methylation-specific PCR is performed prior to the methylation-specific PCR. According to the present invention, at least one of the PCR primers used to amplify the bisulfite-treated template DNA in the first round comprises at least one mismatch with respect to priming (annealing) to the sample template DNA, but nonetheless primes without mismatch to any prior reaction amplified DNA (carry-over contamination) thereby introducing a restriction site suitable for use in selectively degrading the contaminating nucleic acid.

"HeavyMethyl^{™}" as used herein refers to the art-recognized methods described by Cottrell et al. (Nucleic Acids Res. 2004 Jan 13;32(1):e10), comprising the use of blocker oligonucleotides. In the HeavyMethyl^{™} assay, blocking probe oligonucleotides are hybridized to the bisulfite treated nucleic acid concurrently with the PCR *primers.* PCR amplification of the nucleic acid is terminated at the 5' position of the blocking probe, such that amplification of a nucleic acid is suppressed where the complementary sequence to the blocking probe is present. The probes may be designed to hybridize to the bisulfite-treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired. For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivatized at the 3' position with other than a "free" hydroxyl group. For example, 3'-O-acetyl oligonucleotides are representative of a preferred class of blocker molecule. Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide. A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase. Preferably, therefore, the base sequence of said blocking oligonucleotides is required to comprise a sequence having a length of at least 9 nucleotides which hybridizes to the chemically treated nucleic acid sequence, wherein the base sequence of said oligonucleotides comprises at least one CpG, TpG or CpA dinucleotide.

Preferably, real-time PCR assays are performed specified by the use of such primers according to the invention. Real-time PCR assays can be performed with methylation specific primers (MSP-real time) as methylation-specific PCR ("MSP"; as described above), or with non-methylation specific primers in presence of methylation specific blockers (HM real-time) ("HeavyMethyl^{™}", as described above). Real-time PCR may be performed with any suitable detectably labelled probes. For details see below.

In particular aspects of the invention (see EXAMPLE 1 herein below), at least one of the PCR primers used to amplify a bisulfite-treated template DNA comprises at least one mismatch with respect to priming (annealing) to the sample template DNA, but nonetheless primes without mismatch to any prior reaction amplified DNA (carry-over contamination) thereby introducing a restriction site suitable for use in selectively degrading the contaminating nucleic acid..

"MethyLight^{™}" as used herein refers to the art-recognized fluorescence-based real-time PCR technique described by Eads et al (Cancer Res. 59:2302-2306, 1999).. Both MSP or HM methods can be combined with the MethyLight™, which generally increases the specificity of the signal generated in such an assay. Whenever the real-time probe used is methylation specific in itself, the technology is referred to herein as MethyLight™. Another assay, referred to herein as the "QM" (quantitative methylation) assay, also makes use of the methylation specific probe. A methylation unspecific, therefore unbiased real-time PCR amplification is performed which is accompanied by the use of methylation specific probes (MethyLight™) for the methylated and the unmethylated amplificate. That way two signals are generated which can be used to a) determine the ratio of methylated (CG) to unmethylated (TG) nucleic acids, and b) the absolute amount of methylated nucleic acids, when calibrated beforehand.

The MethyLight™ assay is a high-throughput quantitative methylation assay that utilizes fluorescence-based real-time PCR (TaqMan™) technology that requires no further manipulations after the PCR step (Eads et al., Cancer Res. 59:2302-2306, 1999). Briefly, the MethyLight^{™} process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight™ process can by used with a "TaqMan®" probe in the amplification process. For example, double-stranded genomic DNA is treated with sodium bisulfite and subjected to one of two sets of PCR reactions using TaqMan® probes; e.g., with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides. Typical reagents (e.g., as might be found in a typical MethyLight™-based kit) for MethyLight™ analysis may include, but are not limited to: PCR primers for specific bisulfite sequences , i.e. bisulfite converted genetic regions (or bisulfite converted *DNA or bisulfite converted CpG islands);* probes (e.g.TaqMan® or Lightcycler™) specific for said amplified bisulfite converted sequences; optimized PCR buffers and deoxynucleotides; and a polymerase, such as Taq polymerase.

In particular aspects of the invention (see EXAMPLE 1 herein below), at least one of the primers used to amplify a bisulfite-treated template DNA in any of the described real-time PCR assays comprises at least one mismatch with respect to priming (annealing) to the sample template DNA, but nonetheless primes without mismatch to any prior reaction amplified DNA (carry-over contamination) thereby introducing a restriction site suitable for use in selectively degrading the contaminating nucleic acid.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, e.g., matrix assisted laser desorption/ionization mass spectrometry (MALDI) or using electron spray mass spectrometry (ESI).

"MALDI-TOF" as used herein refers to the art-recognized method of Matrix Assisted Laser Desorption/Ionization Mass Spectrometry, which is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapour phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides.

"Array" or "DNA chip" as used herein refers to an arrangement of different oligonucleotides and/or PNA-oligomers bound to a solid phase. The amplificates of various aspect of the present invention may be further detected and/or analysed by means of oligonucleotides constituting all or part of an "array" or "DNA chip." Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

According to additional aspects of the invention, whenever a PCR step is carried out in any of the methods described above, a pre-incubating step will be added, prior to activation of the PCR, by denaturing the usually double-stranded templates, said pre-incubation characterized as enabling sufficient restriction of the potentially carried over PCR product. This is achieved by adding a restriction enzyme, which only cleaves the product in between the primer hybridisation sites, and the according buffer in concentration ranges as exemplified in the examples given.

### EXAMPLE 1

### (A novel method was developed for the carry-over protection in DNA amplification systems based on using primer oligonucleotides in the amplification that comprise one or more mismatches with respect to the sample template DNA, but not with respect to any contaminating (carry-over) nucleic acid)

Aspects of the present invention provide methods for the carry-over protection in DNA amplification systems, the methods comprising using primer oligonucleotides in the amplification that bind to the sample template DNA with one or more mismatches, but bind to any amplifiable contaminant nucleic acid *without* the one or more mismatches.

### RATIONALE:

Presently, there is no robust method available to decontaminate amplification template DNA samples that are compatible when bisulfite-treated DNA is employed as the amplification template in an amplification procedure (*e.g.,* PCR).

The commonly used method, based on use of uracil n-glycosylase (UNG; uracil DNA-glycosylase, UDG), as described above under "BACKGROUND," is based on the incorporation of uridine deoxyribonucleotides during amplification, and therefore any uridine containing, contaminating amplificates can be easily distinguished from the DNA template to be investigated prior to its amplification by digesting with UDG. To this end, UDG is often used to cleave uracil bases from the DNA strands, and therefore render the contaminating amplificate unreadable for the polymerase.

Unfortunately, the problem for decontamination of bisulfite-converted templates is one that cannot be solved by adaptation of the present methods, as any bisulfite-converted DNA will contain uridine as well. Therefore, in any deglycosylase step using UDG, the template DNA would be destroyed along with any contaminating DNA.

To solve this long-standing problem in the art, applicants have developed a novel and surprisingly effective approach that does not rely on any deglycosylase-based approach. The novel methods are based on exploiting differences between any contaminating prior reaction amplificates (*e.g.,* 'carry-over' contamination) and sample template DNA in the reaction to be amplified.

Aspects of the present invention solve this problem by using primer oligonucleotides in the amplification that bind to the sample template DNA with one or more mismatches, but bind to any amplifiable contaminant nucleic acid *without* the one or more mismatches. For example, at least one of these primer oligonucleotides will hybridise to the template DNA only with at least one mismatch ( typically in the middle third of that respective primer). Therefore, when amplification is performed (*e.g.,* PCR) the generated amplificates are slightly different in sequence than the sample template DNA in that the generated amplificates will correspond in sequence to that of the primer oligonucleotides.

In any subsequent amplification, the difference in sequence between this initial first amplification and the sample template DNA used can be exploited. For example, using an enzymatic step that specifically degrades the sequence of the amplified, including the potentially contaminating DNA, only the legitimate sample template DNA will remain intact. Preferably, and typically, the sequence of the amplified DNA will be recognizable by a restriction enzyme. Preferably, the restriction enzyme is selected so that it will only cut sequences within the primers, and that do not occur at other positions, within the sample template DNA, in the region of interest to be amplified.

After degradation of the contaminating DNA, the enzyme is deactivated and will therefore not degrade any products of the subsequent amplification of the legitimate template.

Preferably, the restriction enzyme is selected such that it will only cut DNA that corresponds to a sequence of at least one of the primers, but this sequence will not appear in the template DNA in any region relevant for the amplification. Such differential selection is readily made, for example, in the context of bisulfite treated DNA used as sample template DNA. After bisulfite conversion, the DNA is mainly single-stranded and therefore will not be recognized by most restriction enzymes. Moreover, bisulfite-converted DNA only contains cytosines in positions where it was initially methylated (DNA in humans is only methylated in the sequence context CpG), there are several sequences that cannot occur in bisulfite treated DNA, but can be present in the primer oligonucleotides used for amplification in the mismatched positions. Therefore, aspects of the inventive methods have particularly substantial utility in the context of amplification of bisulfite-treated DNA (and methylation analyses). Alternatively, the decontamination method described here can be employed, and has substantial utility, for any kind of DNA sample for which suitable primers and enzymes can be chosen for the respective region of interest, such that this method provides a surprising effective alternative to the prior art deglycosylase approach.

Therefore, particular aspects provide methods for the carry-over protection in DNA amplification systems, comprising:
- incubating a sample template DNA and a set of at least two primer oligonucleotides with a composition of enzymes and buffers to degrade (*e.g.,* cleave) any contaminating DNA;
- inactivating the composition of enzymes and buffer to preclude, or substantially preclude degrading any product of a subsequent sample template DNA amplification step; and
- amplifying the sample template DNA using the set of primer oligonucleotides and a polymerase, wherein any degraded (*e.g.,* cleaved) contaminating DNA is essentially not amplified, wherein at least one of the primer oligonucleotides hybridizes to at least one of the strands of the contaminating DNA without any mismatches at a selected position, and wherein the same primer binds to the sample template DNA with at least one mismatch at said selected position.

In a *first* step of such embodiments, a set of at least two primer oligonucleotides and a sample template DNA is provided. The primer oligonucleotides are selected such that they are suitable to amplify a template DNA fragment (*e.g.,* region) of interest. Preferably, the primers are designed to amplify the template DNA fragment by means of a polymerase reaction *(e.g.,* a polymerase chain reaction (*e.g.,* PCR)), as known in the art. The primer oligonucleotides are therefore designed to anneal to the template DNA to form a double strand, according to the Watson-Crick base pairing rules, and the length of these oligonucleotide primers are selected such that they anneal at approximately the same temperature. In particularly preferred embodiments, at least one of the primers anneals to the template DNA with a mismatch (that does not follow the Watson Crick base pairing rules). Alternately, both primer oligonucleotides anneal to the sample template DNA with a mismatch. In particularly preferred embodiments, any such mismatch is located in the middle third of the oligonucleotide(s).

Preferably, the mismatch is optimally selected and designed. First, the mismatch is preferably sufficiently distant from the 3'-end of the primer oligonucleotide, such that the extension of the primer by a polymerase is not inhibited to a degree that little or no amplificate from the sample template DNA is produced. Furthermore, to provide for effective decontamination, the mismatch is preferably located sufficiently distant from the 5'-end to ensure that, subsequent to degradation (*e.g.,* annealed primer-mediated cleavage; see further details below), the resultant amplificate would be unable to bind to the primer. Additionally, the mismatch is preferably located in accord with the employed degradation (*e.g*., cleavage) method. For example, where a restriction enzyme is selected for cleavage, the primer position having a mismatch with respect to sample template DNA, would, when paired with its fully complementary strand (*e.g.,* on any contaminating nucleic acid), constitute a functional restriction enzyme recognition and cleavage site (*e.g.,* a self-complementary short sequence as a part of at least one of the primer oligonucleotides). Therefore, such criteria are optimally considered when designing a primer pair.

In a *second* step of such embodiments, a degradation enzyme and matching or compatible buffers are added to achieve degradation (*e.g.,* cleavage) of any contaminating prior reaction amplificates (*e.g.,* to degrade any carry-over contamination) that may be present. Such prior reaction amplificates will, if previously generated using the same primer pair, comprise a sequence (*e.g.,* the self-complementary sequence) that, unlike the corresponding sequence in the sample template DNA, is fully complementary to that introduced into the at least one primer oligonucleotides that was designed to have a mismatch with sample template DNA). Therefore, the sample template DNA is not recognized and cleavable by the enzyme at this step, but amplificate (*e.g.,* DNA) that was generated in preceding amplifications is thereby recognized and removable.

Preferably the degradation enzyme employed in this second step is a restriction enzyme. Preferably, the restriction enzyme is thermolabile, so that it can be effectively heat inactivated prior to amplification of sample template DNA. Preferably, the restriction enzyme is selected such that it recognizes sequences that do not occur in bisulfite-treated DNA. The selected restriction enzyme may be methylation-sensitive or not.

Exemplary useful restriction enzymes include *HaeIII, AluI, CviAII, FatI, NlaIII, MspI, HaII, BfaI, HaeIII, CviJI, HpyCH4V.* In particular embodiments, the restriction enzyme recognizes a sequence selected from the group consisting of GGCC, AGCT, TGCA, GTAC, or CATG. The restriction enzyme is selected so that it does not further cleave any position within the sample template DNA, that is located within the fragment to be amplified by the primer oligonucleotides. Where bisulfite-treated DNA is used as the sample template DNA, the sequences mentioned above should not occur in the sample DNA; they could only occur in amplificates introduced by the primer oligonucleotides, and are therefore characteristic of DNA sequences that were generated in previous amplification round (i.e, characteristic of the contaminating DNA).

In the following tables, an exemplary selection of restriction sites is presented that could be used within the described exemplary methods.

When a bisulfite-treated (sense or antisense) strand serves as the sample template DNA, there are, in the very first round of amplification, no cytosines left within the template other than at methylated CpG positions. Therefore, introduction of one or two dCTPs as mismatches into the forward primer would be particularly advantageous, because a restriction site encompassing such introduced cCTPs will typically not otherwise appear within the amplificate. Likewise, the reverse primer could be advantageously modified to contain one or two guanine bases as mismatch bases, either additionally or alternatively, should it be easier to design it that way. Most primers comprising the sequences listed in the tables below can also be used to hybridise to the first copy of the bisulfite strand, which in bisulfite-treated DNA is usually characterized as being poor in guanines (so-called C-rich strand), and therefore the preferred mismatch modification would be an introduced guanine.

Using particular inventive method as the preferred carry-over protection for MSP (as described in more detail herein below) is somewhat more challenging, because the restriction site must be designed not to interfere with the specificity of the MSP primers. MSP uses primers that specifically bind only to CpGs but not to TpG sites within the priming region. Designing of the primer molecules such that the restriction site overlaps a genomic CpG site, must be avoided as this could lead to the digestion of only one of the two possible amplificates of the bisulfite strands, and to false amplification (however, when it is the aim of the PCR to selectively amplify and detect one methylation state only (for example a CG) in the presence of the other methylation state (for example a TG) no TG sequence will be amplified in the first place and therefore it is not always necessary to also digest sequences which have TG instead of a CG). Therefore combining the present methods with the so-called 'nested MSP' method would likely be the preferred solution.

The conditions for the restriction site are preferably such that the resulting restriction does not unintentionally select for only one methylation state. Therefore it is necessary that the restriction site is not overlapping a position which was a CpG before the bisulfite conversion took place. Furthermore, if the 3'-end nucleotide of the restriction site is the C from such a genomic CpG site or respectively the bisulfite converted T, or the 5'-end nucleotide of the restriction site is the complement of such a C from a CpG (*i.e*., an A or G), the use of this restriction site should be avoided for carry-over protection according to this invention when amplifying bisulfite-treated DNA. However, it is sometimes possible to use enzymes which cleave in both cases, as for example *Apol ,* which cleaves whether the last position in the restriction site is C or T.

Particularly preferred are the shorter restriction sites of the '4-mer cutter' restriction enzymes, as it will be easier to design the appropriate primers.

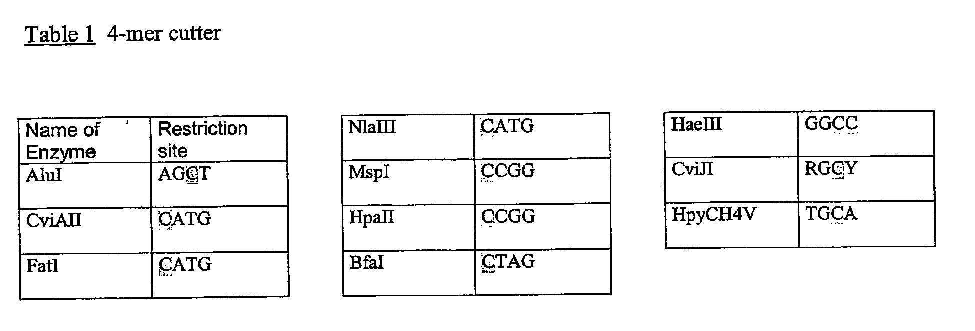

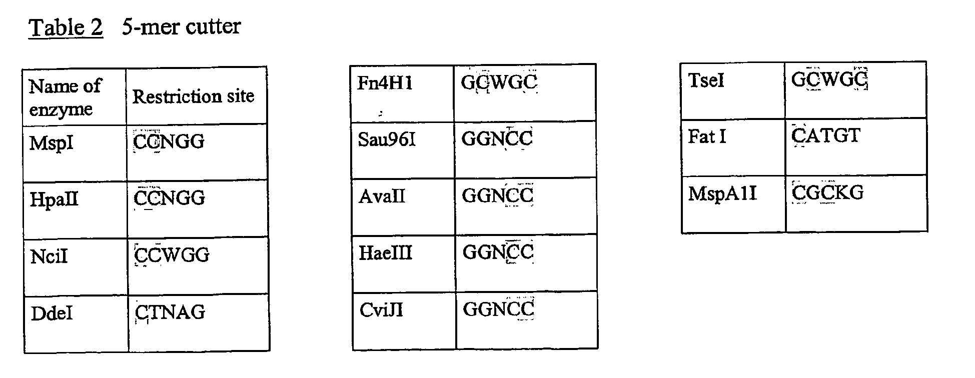

**Table 3 6-mer-cutter**

| Name of enzyme | Restriction site |
|---|---|
| ApoI/XpaI | RAATTY |
| BfrBI | ATGCAT |
| Nsil | ATGCAT |
| HindIII | AAGCTT |
| PciI | ACATGT |
| Af1III | ACRYGT |
| SpeI | ACTAGT |
| BsrI | ACTGGN |
| Bg1II | AGATCT |
| StuI | AGGCCT |
| ScaI | AGTACT |
| NsiI | ATGCAT |
| MfeI | CAATTG |
| BssSI | CACGAG |
| PmlI | CACGTG |
| PvuII | CAGCTG |
| NdeI | CATATG |
| NcoI | CCATGG |
| BseYI | CCCAGC |
| SacII | CCGCGG |
| BsaJI | CCNNGG |
| BtgI | CCRYGG |
| AvrII | CCTAGG |
| StyI | CCWWGG |
| EagI | CGGCCG |
| BsiWI | CGTACG |
| PaeR7I | CTCGAG |
| XhoI | CTCGAG |
| TliI | CTCGAG |
| UbaF5I | CTGATG |
| PstI | CTGCAG |
| BspGI | CTGGAC |
| SfcI | CTRYAG |
| SmlI | CTYRAG |
| BsoBI | CYCGRG |
| AvaI | CYCGRG |
| DrdII | GAACCA |
| EcoRI | GAATTC |
| Zral | GACGTC |
| SacI | GAGCTC |
| SphI | GCATGC |
| NgoMIV | GCCGGC |
| NaeI | GCCGGC |
| BmtI | GCTAGC |
| NheI | GCTAGC |
| Bsp1286I | GDGCHC |
| BamHI | GGATCC |
| SfoI | GGCGCC |
| KasI | GGCGCC |
| NarI | GGCGCC |
| NlaIV | GGNNCC |
| KpnI | GGTACC |
| BanI | GGYRCC |
| BmgI | GKGCCC |
| Bme1580I | GKGCC |
| BanII | GRGCYC |
| BstZ17I | GTATAC |
| SalI | GTCGAC |
| ApaLI | GTGCAC |
| BsiHKAI | GWGCWC |
| NspI | RCATGY |
| BsrFI | RCCGGY |
| BstYI | RGATCY |
| HaeII | RGCGCY |
| SnaBI | TACGTA |
| BspHI | TCATGA |
| BspEI | TCCGGA |
| XbaI | TCTAGA |
| BclI | TGATCA |
| FspI | TGCGCA |
| MscI | TGGCCA |
| BsrGI | TGTACA |
| BsaWI | WCCGGW |
| HaeI | WGGCCW |
| TatI | WGTACW |
| BsaAI | YACGTR |
| EaeI | YGGCCR |

wherein the following is indicated :
**W**=AorT
**R** = A or G
**Y** = C or T
**K**=GorT
**D** = A or T or G
**H** = A or C or T

Generally, however, this invention is applicable to any sample DNA, provided that the fragment of interest does not contain the recognition sequence of the restriction enzyme that is employed.

In additional embodiments, other means are used to cleave any contaminating DNA. Such methods include the cleavage of RNA parts of the primers, formation of loops or other secondary structures and their subsequent cleavage, or any other *motifs* that can be introduced by the primers into the amplificate, but are not a property of the sample template DNA. It is further preferred, that such a motif is also not a property of the primers employed, to avoid their degradation in the enzymatic cleavage step. For example, a primer could be comprised of three regions, a first 5'-region binding to the template DNA and a second region not binding to the template, but being located in the middle of the primer and a third 5'-region binding to the template DNA. The second region should be such that it forms a loop and does therefore not hinder the binding of the primer to the template DNA in a first step. When the temperature reaches a threshold temperature the loop opens up and hence the polymerase will recognize said second region as template and the amplificate will be extended by this region, not originally part of the template DNA, which would now be located within the primer binding site. Such an amplificate could easily be cleaved by an appropriate restriction enzyme, which would leave the primer itself uncleaved, due to its single-stranded nature. Therefore, the inventive enzymatic degradation/cleavage steps will typically have to be specific for double-stranded nucleic acid structures, essentially like the restriction enzymes mentioned above. In one embodiment of the invention, the DNA is cleaved with RNAse H as the enzyme, and primers used comprise ribonucleotides. However, only the double stranded products but not the primers themselves are cleaved by RNAse H, which has been reported to specifically cleave DNA/RNA hybrids.

In a ***third*** step of such embodiments, after enzymatic cleavage, the sample DNA is amplified, while the cleaved contaminating DNA is essentially not amplified. Preferably, this is by means of a polymerase chain reaction (*e.g.*, PCR), but also by other art recognised means, including but not limited to TMA (transcription mediated amplification), isothermal amplifications, rolling circle amplification, ligase chain reaction, and others.

The generated DNA fragments will then be analysed, with respect to their presence, amount, sequence properties, or a combination thereof.

Specific embodiments comprise:
- incubating a sample template DNA and a set of at least two primer oligonucleotides with a composition of enzyme(s) and buffers to cleave any contaminating DNA;
- inactivating or substantially inactivating the composition of enzyme(s) to preclude or substantially preclude degrading/cleaving any product of a subsequent amplification step;
- amplifying the sample template DNA using the set of primer oligonucleotides and a polymerase, wherein any cleaved contaminating DNA is essentially not amplified, wherein at least one of the primer oligonucleotides hybridizes to at least one of the strands of the contaminating DNA without any mismatches at a selected position, and whereing the same primer binds (anneals) to the sample DNA with at least one mismatch at said selected position.

In particular embodiments, the contaminating DNA to be degraded, cleaved, etc. was previously amplified using the same set of primer oligonucleotides. Preferably, the composition of enzymes and buffers comprises at least one restriction endonuclease. Preferably, the restriction endonuclease cleaves specifically at a sequence that is part of a primer oligonucleotide binding site, but does not cleave any sequence of the amplification product generated that is not located within said primer oligonucleotide binding site.

Preferably, in the context of bisulfite-treated sample template DNA, the restriction enzyme cleaves a position that does typically not occur in the bisulfite-treated human genomic DNA.

Inn preferred embodiments, the restriction endonuclease is at least one selected from the group consisting of *HaeIII, AluI, CviAII, FatI, NlaIII, MspI, HpaII, BfaI, HaeIII, CviJI* and *HpyCH4V.*

It is particularly preferred that the composition of degrading/cleavage enzyme(s) and buffer(s) employed is inactivated by heat, and/or by addition of an inhibitor or a change in buffer composition or property (*e.g.*, the pH).

In particularly preferred embodiments, the sample template DNA was previously treated with bisulfite. Preferably, the bisulfite treatment was performed in the presence of a denaturing agent.

Additional aspects provide for use of one of the embodiments to avoid amplification of a contaminating DNA previously amplified from a first sample, in a procedure intended to amplify DNA obtained from a second sample.

Additional aspects provide for use of one of the embodiments to avoid contamination of a diagnostic procedure that relies on the identification of DNA methylation changes in a first sample, the source of said contamination being DNA previously amplified from a second sample using the same set of oligonucleotide primers.

Particularly preferred embodiments provide methods for the detection of cytosine methylation in DNA samples, while not detecting any contaminating DNA derived from other samples that were previously analyzed, comprises:
- treating a genomic DNA sample such that all of unmethylated cytosine bases are converted to uracil, whereas 5-methylcytosine bases remain unchanged, to produce a chemically-treated sample DNA;
- incubating the chemically-treated sample DNA and a set of at least two primer oligonucleotides with a composition of enzymes and buffers suitable to cleave any contaminating DNA but not the chemically-treated sample DNA, wherein at least one of the primer oligonucleotides hybridizes to at least one of the strands of the contaminating DNA without any mismatches at a defined position, and wherein the same primer binds to the sample DNA with at least one mismatch at said position,
- inactivating the composition of enzymes and buffer to preclude or substantially preclude degrading/cleaving any product of a subsequent amplification step;
- amplifying the sample DNA using said set of primer oligonucleotides and a polymerase, wherein any degraded/cleaved contaminating DNA is not amplified or substantially amplified; and
- analyzing the amplified products, wherein the methylation status in the genomic DNA is deduced from the presence of an amplified product and/or from analysis of the sequence within the amplified product.

In a particularly preferred embodiment, the sample DNA is obtained from serum or other body fluids of an individual. It is further particularly preferred, that the DNA samples are obtained from cell lines, tissue embedded in paraffin, for example tissue from eyes, intestine, kidneys, brain, heart, prostate, lungs, breast or liver, histological slides, body fluids and all possible combinations thereof. The term body fluids is meant to comprise fluids such as whole blood, blood plasma, blood serum, urine, sputum, ejaculate, semen, tears, sweat, saliva, lymph fluid, bronchial lavage, pleural effusion, peritoneal fluid, meningal fluid, amniotic fluid, glandular fluid, fine needle aspirates, nipple aspirate fluid, spinal fluid, conjunctival fluid, vaginal fluid, duodenal juice, pancreatic juice, bile, stool and cerebrospinal fluid. It is especially preferred that said body fluids are whole blood, blood plasma, blood serum, urine, stool, ejaculate, bronchial lavage, vaginal fluid and nipple aspirate fluid.

In a particularly preferred embodiment, the chemical treatment is conducted with a bisulfite reagent (= disulfite, hydrogen sulfite). Preferably, the chemical treatment is conducted after embedding the DNA in agarose. Preferably, the chemical treatment is conducted in the presence of a denaturing agent and/or a radical scavenger.

The methylation detection assays described herein above under "DEFINITION," are all preferred embodiments of the invention when performed with the use of said primers according to the invention.

### A fragment of connexin 26 was amplified with primers comprising restriction sites.

The aim of this experiment was to show and confirm, according to particular aspects, that the amplification of bisulfite-treated DNA is not significantly inhibited when primer pairs are employed, which are characterized by specific mismatch positions. The connexin fragment (SEQ ID NO:1; Homo sapiens connexin 26 gene, exon 1. Accession number AF144321) was amplified from human bisulfite-treated PBL DNA. The amplification was performed in a total of 20 µl using the FastStart^{™} Kit for hybridisation probes (Roche, Penzberg), 3.5 mM MgCl₂, 0.3 µM primer (cf. Table 4), 4 µM blocker (SEQ ID NO:2, CCT CTA AAA TAA AAA TTA ACA ATA ACC AAA AAA AAA ACA CCA C-phosphate) and 0.15 µM LC detection probe pair (SEQ ID NO 3, GGA GAA AGA AGC GGG GAt TTC-fluo; SEQ ID NO 4, LCred640-CGG tAt tAG CGG CGt ttt tTt t-pho). 1 ng bisulfite DNA was applied in 10 µl. The amplification was performed in a LightCyler^{™} device (Roche) using the following program: activation 10 min 96°C, and 50 cycles denaturation 10 sec 96°C, annealing 30 sec 56°C, extension 10 sec 72°C. The FRET fluorescence signal was monitored after each annealing step and subsequently analysed with the LC software 3.5 (Roche).

The results are presented in form of Table 4. The first column indicates the primer combinations used; the second and third column list the primer names and sequences; the created restriction site is shaded and the introduced mismatch position is fat printed. The third column lists the names of the restriction enzyme according to these sites, which however have not (yet) been added. The number given in the last column of the table is the cycle threshold number (Ct), when 1 ng of bisulfite-treated DNA was used as a template. A lower Ct indicates a faster amplification than a higher Ct.
//
//

**Table 4: Standard primer with and without introduced restriction site perform similarly well.**

| Primer combination | name | sequence | Restriction enzyme site | Ct 1 ng methylated bisulfit treated template |
|---|---|---|---|---|
| 1 | 6211.4F1 | GGTATATTGTTGAAAGTAATTGAATAAAAT (SEQ ID NO:5) | none | 36.1 |
| 1 | 6211.4R1 | AAACAATACCCTCTAAAATAAAAATTAAC (SEQ ID NO:6) | none | |
| 2 | 6211.4F-Alu | GGTATATTGTTGAAAGCTAATTGAATAAAAT (SEQ ID NO:7) | Alul | 36.9 |
| 2 | 6211.4R-Bfa-1 | AAACAATACCCTCTAGAAATAAAAATTAAC (SEQ ID NO:8) | Bfal | |
| 3 | 6211.4F-Hpy | GGTATATTGTTGAAAGTAATTGCATAAAAT (SEQ ID NO:9) | HpyCH4 V | 36.5 |
| 3 | 6211.4R-Bfa-2 | AAACAATACCCTCTAGAATAAAAATTAAC (SEQ ID NO:10) | Bfal | |

Primer combination 1 is the standard primer pair used so far in routine analysis. It can be seen from the Table 4 that the modified primer (combinations 2 and 3) performed similarly well when amplifying the same connexin 26 fragment using the same methylated bisulfite-treated template DNA. In comparison to the unmodified primer (combination 1: 6211.4F1/6211.4R1), the other 2 primer combinations show a only slightly reduced performance in the PCR (see Table 4).

### A fragment of connexin 26 was amplified with primers comprising restriction sites using a pre-incubation with and without restriction enzyme.

The aim of this experiment was to show and confirm, according to particular aspects, that the pre-incubation of sample template nucleic acids and the presence of restriction enzymes and restriction buffer does not inhibit the amplification of template DNA in a PCR with said primers. Different connexin 26 PCRs were performed as specified above with primer pairs 6211.4F-Alu/6211.4R-Bfa-1 (combination 2); 6211.4F-Hpy/6211.4R-Bfa-2 (combination 3). In contrast to the experiment described above, a 30 min incubation at 37°C was performed immediately before the PCR program started with an activation step. For control purpose this pre-incubation step was performed prior to each PCR, in the same tube, once with the required restriction enzymes added to the PCR mix and once without. Prior to the PCR with primer combination 2 (6211.4F-Alu/6211.4R-Bfa-1), the tube was pre-incubated with added restriction enzymes *AluI* and *BfaI* (New England Biolabs), prior to the PCR with primer combination 3 (6211.4F-Hpy/6211.4R-Bfa-2, the tube was pre-incubated with added restriction enzymes *BfaI* and *HpyCH4 V* (New England Biolabs). Each restriction enzyme was applied in an amount of 2 U in the amplification reaction.

The results are presented in Table 5. The first and second columns indicate the primer combinations used; the third column lists the restriction enzymes used. The numbers given in the last two columns of the table are the cycle threshold numbers (Ct), when (column 4) 9000 copies of the PCR product were present or (column 5) 1 ng of bisulfite-treated DNA was used as a template. A lower Ct indicates a faster amplification than a higher Ct.

The results show, that the restriction enzymes did not significantly reduce the PCR efficiency of the tested primer combination on human bisulfite-treated template DNA (see column 5). However, if PCR product generated with these primer pairs beforehand was used as template DNA (*i.e*., the potentially contaminating DNA), the pre-incubation with respective restriction enzymes caused an increase of the cycle thresholds of up to nearly 4 cycles (see column 4) with primer combination 3

By comparing the Ct values in column 4 and 5, the effect of adding the enzymatic digest pre-incubation step results in a reduced Ct value more significantly when the `contaminant' was used as a template.
//
//
//

**Table 5: Primer with and without introduced restriction site.**

| Primer combination | Primer name | Restriction enzyme | Ct 9000 copies of PCR product | Ct on 1 ng methylated bisulfit treated template |
|---|---|---|---|---|
| 3 | 6211.4F-Hpy /6211.4R-Bfa-2 | none | 28.9 | 35.0 |
| 3 | 6211.4F-Hpy /6211.4R-Bfa-2 | HpyCH4 V/ Bfal | 32.5 | 34.4 |
| 2 | 6211.4F-Alu/6211.4R-Bfa-1 | none | 29.1 | 35.1 |
| 2 | 6211.4F-Alu/6211.4R-Bfa-1 | Alul/Bfal | 29.5 | 35.5 |

### EXAMPLE 2

### (A novel method was developed for the carry-over protection in DNA amplification systems based on pre-incubation with a restriction enzyme)

Aspects of the present invention provide methods for the carry-over protection in DNA amplification systems, the methods comprising pre-incubation with a restriction enzyme.

Particular aspects provide a method for the specific amplification of single-stranded sample template DNA in the presence of potentially contaminating double-stranded PCR products from previous amplification experiments (carry-over contamination). The method comprises adding a pre-incubation step prior to the intended PCR amplification. During this pre-incubation, a specific restriction digest is performed by a preferably thermolabile restriction enzyme, selectively cleaving the double-stranded prior reaction PCR product, but not the single-stranded sample template nucleic acid. Any contaminating DNA is subsequently degraded or at least fragmented enzymatically and only the sample DNA is amplified in the next step. The method is useful for the decontamination of single-stranded DNA samples or rather the inhibition of amplification of 'carry over products,' in particular in the context of DNA methylation analysis.

### RATIONALE:

Presently, there is no robust method available to decontaminate amplification template DNA samples that are compatible when bisulfite-treated DNA is employed as the amplification template in an amplification procedure (*e.g.,* PCR).

The commonly used method, based on use of uracil n-glycosylase (UNG; uracil DNA-glycosylase, UDG), as described above under "BACKGROUND," is based on the incorporation of uridine deoxyribonucleotides during amplification, and therefore any uridine containing, contaminating amplificates can be easily distinguished from the DNA template to be investigated prior to its amplification by digesting with UDG. To this end, UDG is often used to cleave uracil bases from the DNA strands, and therefore render the contaminating amplificate unreadable for the polymerase.

Unfortunately, the problem for decontamination of bisulfite-converted templates is one that cannot be solved by adaptation of the present methods, as any bisulfite-converted DNA will contain uridine as well. Therefore, in any deglycosylase step using UDG, the template DNA would be destroyed along with any contaminating DNA.

To solve this problem, applicants have developed a novel and surprisingly effective approach that does not rely on any deglycosylase-based approach. The novel methods are based on exploiting differences between the contaminating amplificates and the sample template DNA.

Aspects of the present invention provide a method comprising:
- incubating the sample DNA and a set of at least two primer oligonucleotides with a composition of enzymes and buffers to degrade (*e.g.,* cleave) any contaminating DNA;
- inactivating the composition of enzymes and buffer to preclude degrading (*e.g.,* cleaving) or substantially degrading any product of a subsequent amplification step; and
- amplifying the sample DNA using the set of primer oligonucleotides and a polymerase, wherein any degraded/cleaved contaminating DNA is essentially not amplified.

Therefore, according to aspects of the present invention, with respect to any subsequent amplification (*i.e*., amplification of a sample template DNA), the difference between a prior contaminating amplification product and the template DNA used can be exploited in any enzymatic step that specifically degrades the double stranded nucleic acids of the amplified, potentially contaminating DNA, while allowing the legitimate template DNA to remain intact.

After degradation of the contaminating DNA, the enzyme is deactivated and will therefore not degrade any products of the subsequent amplification of the legitimate template.

Preferably, the enzyme employed in the pre-incubation step is a restriction enzyme. It is further preferred that the restriction enzyme is thermolabile. Preferably, the restriction enzyme, recognizes sequences that do occur in bisulfite-treated DNA. For the present invention, it is not important whether the selected restriction enzyme is methylation sensitive or not.

The conditions for the restriction site are preferably such that the resulting restriction does not unintentionally select for only one methylation state. Therefore it is necessary that the restriction site is not overlapping a position which was a CpG before the bisulfite conversion took place. Furthermore, if the 3'-end nucleotide of the restriction site is the C from such a genomic CpG site or respectively the bisulfite converted T, or the 5'-end nucleotide of the restriction site is the complement of such a C from a CpG (*i.e.,* an A or G) the use of this restriction site should be avoided for carry over protection according to this invention when amplifying bisulfite-treated DNA. In some instances, it is however possible to use enzymes which cleave in both cases, as for example *Apol,* which cleaves whether the last position in the restriction site is C or T.

The present aspects are particularly useful for the analysis of methylation in DNA, because after a treatment such as a bisulfite-treatment the template DNA is single-stranded, whereas the PCR product is double-stranded.

Generally, however, this invention is applicable to any single-stranded sample DNA, provided that the fragment of interest does contain a recognition sequence of the restriction enzyme that is employed.

It is particularly preferred that the composition of enzyme(s) and buffer(s) employed in the pre-incubation step is inactivatable by heat, and/or by addition of an inhibitor or a change in buffer composition or property (*e.g.,* the pH).

In a particularly preferred embodiment, the sample template DNA was previously treated with bisulfite. It is preferred that the bisulfite treatment was performed in the presence of a denaturing agent.

Additional aspects provide for use of one of the embodiments to avoid amplification of a contaminating DNA previously amplified from a first sample, in a procedure intended to amplify DNA obtained from a second sample.

Further aspects provide for use of one of the embodiments to avoid contamination of a diagnostic procedure that relies on the identification of DNA methylation changes in a first sample, the source of said contamination being DNA previously amplified from a second sample using the same set of oligonucleotide primers.

Particularly preferred embodiments provide methods for the detection of cytosine methylation in DNA samples, while not detecting any contaminating DNA derived from other samples that were previously analyzed, comprising:
- treating (*e.g.,* chemically) a genomic DNA sample such that all unmethylated cytosine bases are converted to uracil, while 5-methylcytosine bases remain unchanged, producing a treated (*e.g.,* chemically treated) sample DNA;
- incubating the treated sample DNA and a set of at least two primer oligonucleotides with a composition of enzymes and buffers to degrade (*e.g.,* cleave) any contaminating DNA, but not the treated single-stranded sample DNA;
- inactivating the composition of enzymes and buffer to preclude or substantially preclude degrading (*e.g.,* cleaving) any product of the subsequent amplification step;
- amplifying the sample DNA using said set of primer oligonucleotides and a polymerase, wherein any cleaved contaminating DNA is essentially not amplified; and
- analyzing the amplified products, wherein the methylation status in the genomic DNA is deduced from the presence of an amplified product and/or from the analysis of the sequence within the amplified product.

In a particularly preferred embodiment, the sample DNA is obtained from serum or other body fluids of an individual. It is further particularly preferred, that the DNA samples are obtained from cell lines, tissue embedded in paraffin, for example tissue from eyes, intestine, kidneys, brain, heart, prostate, lungs, breast or liver, histological slides, body fluids and all possible combinations thereof. The term body fluids is meant to comprise fluids such as whole blood, blood plasma, blood serum, urine, sputum, ejaculate, semen, tears, sweat, saliva, lymph fluid, bronchial lavage, pleural effusion, peritoneal fluid, meningal fluid, amniotic fluid, glandular fluid, fine needle aspirates, nipple aspirate fluid, spinal fluid, conjunctival fluid, vaginal fluid, duodenal juice, pancreatic juice, bile, stool and cerebrospinal fluid. It is especially preferred that said body fluids are whole blood, blood plasma, blood serum, urine, stool, ej aculate, bronchial lavage, vaginal fluid and nipple aspirate fluid.

In a particularly preferred embodiment, the chemical treatment is conducted with a bisulfite reagent (*e.g*., = disulfite, hydrogen sulfite). Preferably, the chemical treatment is conducted after embedding the DNA in agarose, or that it is conducted in the presence of a denaturing agent and/or a radical scavenger.

The methylation detection assays described herein above under "DEFINITIONS," are all preferred embodiments of the invention when performed with the use of said primers according to the invention.

### Amplification of a fragment of connexin 26 in a HeavyMethyl assay as described above after pre-incubation with and without a restriction enzyme cleaving the amplified fragments in between the two primer binding regions.

The aim of this experiment was to show that the amplification of bisulfite-treated DNA is not inhibited, whereas the amplification of potentially contaminating 'carry over amplificates' is significantly inhibited, when performing a restriction digest prior to the PCR start by activation of the PCR at elevated temperatures.

The connexin fragment (SEQ ID NO:1; *Homo sapiens* connexin 26 gene, exon 1. Accession number AF144321) was amplified from human bisulfite-treated PBL DNA. The amplification was performed in a total of 20 µl using the FastStart^{™} Kit for hybridisation probes (Roche, Penzberg), 3,5 mM MgCl₂, 0.3 µM primer (cf. Table 1), 4 µM blocker (SEQ ID NO:2, CCTCTAAAATAAAAATTAACAATAACCCACCAC-phosphate) and 0.15 µM LC detection probe pair (SEQ ID NO:3, GGAGAAAGAAGCGGGGAtTTC-fluo; SEQ ID NO:4, LCred640-CGGtAttAGCGGCGtttttTtt-pho). 1 ng bisulfite DNA was applied in 10µl. The amplification was performed in a LightCyler^{™} device (Roche) using the following program: activation 10 min 96°C, and 50 cycles denaturation 10 sec 96°C, annealing 30 sec 56°C, extension 10 sec 72°C. The FRET fluorescence signal was monitored after each annealing step and subsequently analysed with the LC software 3.5 (Roche).

Prior to the activation step, the amplification reactions were incubated for 30 min at 37°C once without and once with restriction enzymes (*XpaI (ApoI)and HphI;* 2 U each; New England Biolabs). These restriction enzymes were selected as specifically cleaving the amplified product, but leaving the region of the primer binding site intact. The FRET fluorescence signal was monitored after each annealing step and subsequently analysed with the LC software 3.5 (Roche).

The results are presented in Table 1. The first and second columns present the names and sequences of the primers used; the third column lists the names of the restriction enzymes used during pre-incubation. The numbers given in the last two columns of the table are the cycle threshold numbers (Ct), when (column 4) 9000 copies of the PCR product were present or (column 5) 1 ng of bisulfite-treated DNA was used as a template. A lower Ct indicates a faster amplification than a higher Ct.

It could therefore be demonstrated, that pre-incubation with restriction enzymes which cleave the double-stranded PCR product did not significantly reduce the PCR efficiency on human bisulfite-treated template DNA (see column 5, comparing first with second row). However, if PCR product was used as template DNA (column 4), the pre-incubation with these restriction enzymes caused a significant increase of the cycle thresholds of up to 10 cycles. Thereby an inhibitory effect is achieved that is mirrored in a reduction of the copy-number of the potentially contaminating DNA by a factor of 1000 (2¹⁰ = 1024).

**Table 1: Amplification of contaminating PCR product can be inhibited as is shown for connexine26 HeavyMethyl PCR with and without pre-incubation with restriction enzymes (fragment internal restriction site).**

| Primer name | Primer sequence | Restriction enzyme | Ct 9000 copies of PCR product | Ct on 0.1 ng methylated bisulfit treated template |
|---|---|---|---|---|
| 6211.4F1 | GGTATATTGTTGAAAGTAATTGAATAAAAT (SEQ ID NO:5) | none | 29 | 38.2 |
| 6211.4R1 | AAACAATACCCTCTAAAATAAAAATTAAC (SEQ ID NO:6) | | | |
| 6211.4F1 | GGTATATTGAAAGTAATTGAATAAAAT (SEQ ID NO:11) | Xpal /Hph I | 39 | 38.6 |
| 6211.4R1 | AAACAATACCCTCTAAAATAAAAATTAAC (SEQ ID NO:12) | | | |

To demonstrate the location of the restriction sites, the sequence of the amplificate is given in Figure 11 (see SEQ ID NO:13).

These alternative restriction recognition sites which are located within the amplificate can also be used for carryover protection according to the invention and are especially suitable in this example.

**Table 2 *^{,}**:**

| cleaving position within fragment | enzyme | recognition sequence |
|---|---|---|
| 17 | Tsp509l | ^AATT |
| 34 | Tsp5091 | ^AATT |
| 34 | Apol / Xbal | R^AATT_Y |
| 65 | Mnll | CCTCNNNNNN_N^ |
| 73 | Hphl | GGTGANNNNNNN_N^ |
| 131 | Msel | T^TA_A |
| 132 | Tsp509l | ^AATT |
| 139 | Mnll | CCTCNNNNNN_N^ |

| | | |
|---|---|---|
| *Wherein the symbol ^ indicates cleavage at the one strand and the symbol _ indicates cleavage of the other. ** Wherein **R** indicates A or G and **Y** indicates C or T and **N** indicates any nucleotide | | |

### EXAMPLE 3

### (A novel method was developed for the carry-over protection in DNA amplification systems based on the incorporation of ribonucleotide-triphosphates)

Aspects of the present invention provide methods for the carry-over protection in DNA amplification systems, the methods comprising: incorporation of ribonucleotide-triphosphates during amplification to produce chimeric amplificates; and use of chimeric amplificate-specific enzymes for specifically degrading any carry-over amplificate contamination, while leaving subsequent sample templates in tact.

### RATIONALE:

Presently, there is no robust method available to decontaminate amplification template DNA samples that are compatible when bisulfite-treated DNA is employed as the amplification template in an amplification procedure (*e.g*., PCR).

The commonly used method, based on use of uracil n-glycosylase (UNG; uracil DNA-glycosylase, UDG), as described above under "BACKGROUND," is based on the incorporation of uridine deoxyribonucleotides during amplification, and therefore any uridine containing, contaminating amplificates can be easily distinguished from the DNA template to be investigated prior to its amplification by digesting with UDG. To this end, UDG is often used to cleave uracil bases from the DNA strands, and therefore render the contaminating amplificate unreadable for the polymerase.

Unfortunately, the problem for decontamination of bisulfite-converted templates is one that cannot be solved by adaptation of the present methods, as any bisulfite-converted DNA will contain uridine as well. Therefore, in any deglycosylase step using UDG, the template DNA would be destroyed along with any contaminating DNA.

To solve this problem, applicants have developed a novel and surprisingly effective approach that does not rely on any deglycosylase-based approach. The novel methods are based on exploiting differences between contaminating prior amplificates and template DNA in the reaction to be amplified.

Particular aspects of the present invention provide a method to reduce contamination by carry-over of PCR products, comprising use of ribonucleotides which are present during the amplification step and which are incorporated in the copied strand by a polymerase (*e.g*., heat-stable polymerase). In particular embodiments, the ribonucleotides used are adenosine-triphosphate and/or uridine-triphosphate, although others can be used (*e.g*, rATP, rUTP, rTTP, rCTP, and rGTP). At least one ribonucleotide is incorporated into the amplificate. Therefore, when nucleic acid amplification is performed, for example by PCR, the generated amplificates are different (because of the present of ribonucleotides) than the template DNA. Preferably they differ in terms of sensitivity towards RNase digestion, based on the fact that they will comprise ribonucleotides.

In any subsequent amplification, the difference in the type of nucleotides incorporated between this first amplificate and the template DNA used in a subsequent amplification can be exploited to preclude carry-over contamination of chimeric amplificates. According to particular aspects, in any enzymatic step that specifically degrades the type of polymeric nucleic acid of the amplified, potentially contaminating DNA, only the legitimate template DNA will remain intact (will remain further amplifiable by the primers). Typically, the different nature of the nucleic acid of the amplified DNA will be recognized by a RNA degrading or fractionating enzyme, such as a RNase. This RNAse will only cut nucleic acids (double- and/or single-stranded) that contain ribonucleotides or chimeric (as defined herein above) nucleic that contain a mixture of deoxyribonucleotides and ribonucleotides.

After degradation of any contaminating DNA (carry-over contamination) prior to amplification, the RNA degrading enzyme is deactivated (*e.g*., by heat, etc.) and thus will not degrade any amplification products of the subsequent amplification of the legitimate template;

In alternate embodiments, any chimeric amplificate, if present as a contaminant in a subsequent amplification reaction, will be fragmented. Newly synthesized chimeric nucleotide products are cleaved at the incorporated ribonucleotides, providing a population of fragments not suitable for further amplification with the same primer set.

Exemplary enzymes suitable for this purpose are described in U.S. Patent No. 5,939,292 to Gelfand et al., incorporated by reference herein. For example, having utility for the present purposes are recombinant thermostable DNA polymerases that are mutant forms of a naturally occurring thermostable DNA polymerase, wherein said naturally occurring thermostable DNA polymerase has an amino acid sequence comprising amino acid sequence motif SerGlnIleGluLeuArgXaa, wherein "Xaa" at position 7 of said sequence motif is a valine residue (Val) or an isoleucine residue (Ile); wherein said mutant form has been modified to contain an amino acid other than glutamic acid (Glu) at position 4 of said sequence motif; and wherein said mutant form possesses reduced discrimination against incorporation of an unconventional nucleotide in comparison to said naturally occurring thermostable DNA polymerase. In particular embodiment The underlying naturally-occurring DNA polymerase (that has been mutatated as described above) is at least one selected from the group consisting of *Thermus aquaticus, Thermus caldophilus, Thermus chliarophilus, Thermus filiformis, Thermus flavus, Thermus oshimai, Thermus ruber, Thermus scotoductus, Thermus silvanus, Thermus species Z05, Thermus species sps17, Thermus thermophilus, Thermotoga maritima, Thermotoga neapolitana, Thermosipho africanus, Anaerocellum thermophilum, Bacillus caldotenax,* and *Bacillus stearothermophilus* DNA polymerases. In particular aspects the mutant polymerase is based upon a naturally occurring DNA polymerase from a thermostable *Thermotoga* or *Thermus* species. In particular aspects the mutant polymerase is based on a naturally occurring DNA polymerase comprising the amino acid sequence LeuAspTyrSerGlnIleGluLeuArgValLeuAla HisLeuSer.

Particular aspects provide a method to eliminate or preclude carry-over contamination, comprising: (i) incorporating at least one ribonucleoside-triphosphate (rATP, rUTP, rTTP, rCTP or rGTP) in all PCR products or other amplified products (by substituting at least one deoxy-ribonucleotide with the respective ribonucleotides); and (ii) treating all subsequent fully preassembled starting reactions with a degrading, cleaving or fragmenting enzyme (*e.g.,* a RNase), followed by inactivation (*e.g.,* thermal) of said enzyme prior to amplification in the subsequent reactions. The degrading, cleaving or fragmenting enzyme (*e.g.,* RNase) cleaves the previously amplified fragment, which is characterized as carrying at least one ribonucleotide instead of a deoxy-ribonucleotide. In the case of RNAse, there is hydrolyses of the phosphodiester bond between a ribonucleotide and the deoxynucleotide attached to this residue, but little or no affect on natural or bisulfite-treated (*i.e*., only deoxynucleotide containing) DNA (or on ribonucleotide reactants). The resulting degraded, digested or fragmented products cannot further serve as template for a template-dependent polymerase chain reaction. Because the enzyme (*e.g.,* RNase) is inactivated (*e.g.,* by heat denaturation) prior to the actual amplification (*e.g.,* PCR), carry-over contamination of PCRs can be effectively controlled or eliminated where the contaminants contain ribonucleotides in place of deoxynucleotides.

In a first step of particular embodiments, a pair of primer oligonucleotides, NTPs and dNTPs, a ribonucleotide tolerant polymerase and a sample DNA template is provided. Thereafter, any contaminating DNA is degraded enzymatically (*e.g.,* RNase), and only the sample DNA serves as template in the final amplification step. The method is useful for the decontamination of DNA samples, and in particular in the context of DNA methylation analysis (*e.g.,* using bisulifte-treated DNA as described herein above).

In preferred embodiments, a heat-stable polymerase is used, the heat-stable polymerase suitable to incorporate ribonucleotides as well as deoxynucleotides into the amplficate. Exemplary, suitable heat-stable polymerases are, as discussed herein above in more detail, described in detail in US patent 5,939,292 to Gelfand et al.

A rNTP:dNTP ratio of 1:1 or less, in combination with the suitable heat-stable polymerases, is sufficient for the present purposes. In particular embodiments of the invention, the rNTP:dNTP ratio is reduced to less than 1:8. In particular embodiments, the ratio may be as low as 1:15, 1:20, 1:25. 1:50, 1:80, 1:100 or 1:200, depending on the particular experimental design and desired length of fragments. In preferred embodiments of the invention, the concentration of the at least one ribonucleotide in the reaction mixture is less than the concentration of the corresponding deoxyribonucleotide (*e.g.,* the rNTP:dNTP ratio is 1:1 or less). I, other embodiments the rNTP:dNTP ratio is greater than 1:1.

The choice of the contaminant (chimeric amplificate)-degrading or fractioning enzyme mentioned above is a key step of the invention. Preferably, the enzyme selected for degrading, digesting or fragmenting only cleaves DNA that contains at least one ribonucleotide (i.e., a previously generated contaminating chimeric amplificate). Prior to amplification, the desired template does not contain ribonucleotides, and is thus protected from enzymatic digestion at this step.

Significantly, even though bisulfite-treated DNA contains uracil bases instead of unmethylated cytosine bases, these bases are deoxy-nucleosides, and therefore protected from RNase digestion. Additionally, after bisulfite conversion, the DNA is mainly single-stranded and therefore will not be recognized by those RNA digesting enzymes that are specific for double-stranded nucleic acids. Therefore, the method can be most easily employed for bisulfite treated DNA, when using a double strand specific fractionating or degrading enzyme (*e.g.,* a double-strand-specific RNase). However, in view of potential remaining single-stranded contaminants within the reaction tube, particular embodiments use a degrading, digesting or fragmenting enzyme that cleaves double-stranded as well as single-stranded nucleic acids or a combination of two enzymes, one being specific for single-stranded and one being specific for double-stranded nucleic acids comprises ribonucleotides.

In preferred embodiments, the composition of degrading, digesting or fragmenting enzyme enzyme(s) and buffer(s) employed is inactivated by heat, by addition of an inhibitor, a change in buffer composition or property (*e.g.,* pH), or combinations thereof.

According to additional aspects, the novel decontamination methods described here have utility with respect to essentially any kind of DNA sample, and serves as an surprising effective alternative to the deglycosylase approach that is widely used in the art, particularly for bisulfite-treated DNA templates in the context of methylation analysis where the deglycosylase approach is not useable, as discussed herein above.

As stated above, in the first step of particular embodiments, a set of at least two primer oligonucleotides and a sample DNA is provided. The primer oligonucleotides are chosen such that they amplify a fragment of interest. Preferably, these primers are designed to amplify a DNA fragment of a template DNA sample by means of a polymerase reaction, and in particular a polymerase chain reaction, as is known in the art. The primer oligonucleotides are therefore designed to anneal to the template DNA to form a double strand, following the Watson-Crick base pairing rules, and the length of these oligonucleotide primers will be selected such that they anneal at approximately the same temperature. Perfect complementarity is not necessary required, but the primers must anneal specifically and suitably for allowance of primer extension/template amplification.

In the second step of exemplary embodiments, a degrading, digesting or fragmenting enzyme and compatible buffers are added to achieve cleavage of any contaminating amplificates that were generated in any preceding amplification reactions. Such prior amplificates will have the property that, being generated with the same primer pair in the presence of ribonucleotides, they would comprise ribonucleotides. As the enzyme employed in this second step will degrade, fragment or cleave specifically only those nucleic acids which comprise ribonucleotides, the sample template (*e.g.,* template DNA) would not be recognized and cleaved by the enzyme at this step, and only the contaminating DNA, and not the sample template, is removed before amplification.

Preferably, the enzyme employed in this second step is a RNase. It is further preferred that the RNase is thermolabile. Preferably the enzyme employed in this second step recognizes hybrid nucleic acids that do not occur in bisulfite treated DNA. Preferably, a thermolabile RNase is used that recognizes hybrid nucleic acids that do not occur in bisulfite-treated DNA.

Generally, however, aspects of the present invention are applicable to essentially any sample DNA, provided that the RNA degrading or fractionating enzyme employed in the first step is specific enough to selectively cleave any prior amplified carry-over product comprising at least one ribonucleotide.

In a particular embodiment, the amplified nucleic acids are cleaved with RNAse H as the enzyme. RNAse H, which is specific to cleave DNA/RNA hybrids (and chimeric nucleic acids as defined herein), cleaves only any double-stranded prior amplified carry-over products comprising at least one ribonucleotide, but not the single-stranded ample template nucleic acids.

In a third step of exemplary embodiments, after, e.g., enzymatic degrading, digesting, cleavage, or fragmenting, the sample DNA is amplified, while the cleaved or degraded contaminating DNA is essentially not amplified. This can be done, in a particularly preferred embodiments of the invention, by means of a polymerase chain reaction (PCR), but also by other means of DNA amplification known in the art, like TMA (transcription mediated amplification), isothermal amplifications, rolling circle amplification, ligase chain reaction, and others.

The generated DNA fragments will then be analysed, concerning their presence, the amount, or their sequence properties or a combination thereof.

In particularly preferred embodiments of the invention, the sample DNA is previously treated with bisulfite. Preferred, the bisulfite treatment was performed in the presence of a denaturing agent.

Additional embodiments provide methods for precluding amplification of a contaminating DNA previously amplified from a first sample, in a procedure intended to amplify DNA obtained from a second sample.

Additional embodiments provide methods for precluding contamination of a diagnostic procedure that relies on the identification of DNA methylation changes in a first sample, the source of said contamination being DNA previously amplified from a second sample using the same set of oligonucleotide primers.

Particular aspects provide methods for methylation analysis (detection of cytosine methylation in DNA samples), comprising:
- treating (*e.g.,* chemically) a genomic DNA sample to that all unmethylated cytosine bases are converted to uracil, while the 5-methylcytosine bases remain unchanged, producing a treated sample DNA;
- incubating the treated sample DNA and a set of at least two primer oligonucleotides with a composition of enzymes and buffers to cleave any contaminating DNA, but not the treated sample DNA, wherein said composition of enzymes includes a ribonucleotide-tolerant polymerase and an enzyme capable of specifically degrading or fractionating nucleic acids consisting of ribonucleotides and deoxynucleotides, or nucleic acids comprising ribonucleotides;
- inactivating the composition of enzymes and buffer to preclude substantially cleaving any product of a subsequent amplification step;
- amplifying the sample DNA using said set of primer oligonucleotides and said polymerase, wherein any degraded, digested, cleaved, fragmented, etc., contaminating DNA is not amplified,
- determining, based on the amplificate (*e.g.,* the presence of an amplified product and/or the sequence within the amplified product), the methylation status in the genomic DNA is deduced from.

In particular embodiments, the sample DNA is obtained from serum or other body fluids of an individual. In particular embodiments, the sample DNA is obtained from cell lines, tissue embedded in paraffin, for example tissue from eyes, intestine, kidneys, brain, heart, prostate, lungs, breast or liver, histological slides, body fluids and all possible combinations thereof. The term body fluids is meant to comprise fluids such as whole blood, blood plasma, blood serum, urine, sputum, ejaculate, semen, tears, sweat, saliva, lymph fluid, bronchial lavage, pleural effusion, peritoneal fluid, meningal fluid, amniotic fluid, glandular fluid, fine needle aspirates, nipple aspirate fluid, spinal fluid, conjunctival fluid, vaginal fluid, duodenal juice, pancreatic juice, bile, stool and cerebrospinal fluid. It is especially preferred that said body fluids are whole blood, blood plasma, blood serum, urine, stool, ejaculate, bronchial lavage, vaginal fluid and nipple aspirate fluid.

In particularly preferred embodiments, the treatment is chemical treatment conducted with a bisulfite reagent (= disulfite, hydrogen sulfite). In particular aspects, the chemical treatment is conducted after embedding the DNA in agarose, or conducted in the presence of a denaturing agent and/or a radical scavenger.

The methylation detection assays described herein above under "DEFINITIONS," are all preferred embodiments of the invention when performed with the use of said primers according to the invention.

## Claims

1. A method for elimination of carry-over contamination in nucleic acid amplification reactions, in which the template is a bisulfite-treated nucleic acid, comprising the steps of:
a) amplifying a first bisulfite-treated nucleic acid template in order to provide a first nucleic acid amplificate in a first nucleic acid amplification reaction; and
b) prior to a second amplification, in which the template is another bisulfite-treated nucleic acid, adding a degrading enzyme suitable to degrade any contaminating first nucleic acid amplificate from said first nucleic acid amplification reaction to reactants of said second nucleic acid amplification reaction;
c) inactivating said degrading enzyme; and
d) amplifying the bisulfite-treated nucleic acid template of the second nucleic acid amplification reaction in order to provide a second nucleic acid amplificate lacking or substantially lacking any contaminating amplificate,
(i) wherein said degrading enzyme is a sequence specific restriction enzyme,
(ii) wherein in each nucleic acid amplification reaction at least one primer oligonucleotide is used that binds to the sample template with one or more mismatches, but binds to any amplifiable contaminant nucleic acid without the one or more mismatches, thereby introducing a restriction site into said first and contaminating nucleic acid.
(iii) wherein the same set of primer oligonucleotides is used in each amplification reaction.

2. The method of claim 1, wherein said degrading enzyme specifically degrades the double-stranded nucleic acids that hybridized to the primer oligonucleotide without forming a mismatch.

3. The method of claim 1 or 2, wherein said degrading enzyme is a thermolabile restriction enzyme and wherein the nucleic acid template sequence does not comprise the recognition sequence of said restriction enzyme.

4. The method of claim 3, wherein said restriction enzyme does not cleave at a position within the fragment to be amplified.

5. The method of claim 3, wherein said restriction enzyme recognizes sequences that do not occur in bisulfite treated DNA.

6. The method of claim 3, wherein said restriction enzyme recognition site comprises one of the sequences from the group consisting of GGCC, AGCT, TGCA, GTAC, and CATG.

## Patentansprüche

1. Verfahren zur Eliminierung von Übertragungskontamination bei Reaktionen zur Amplifizierung von Nukleinsäuren, wobei als Vorlage eine mit Bisulfit behandelte Nukleinsäure ist, umfassend die Schritte:
a) Amplifizieren einer ersten mit Bisulfit behandelten Nukleinsäurevorlage zum Bereitstellen eines ersten Nukleinsäureamplifikats in einer ersten Reaktion zur Amplifizierung von Nukleinsäuren; und
b) vor einer zweiten Amplifizierung, wobei es sich bei der Vorlage um eine weitere mit Bisulfit behandelten Nukleinsäure handelt, Zugabe eines Abbauenzyms, das geeignet ist, jedes kontaminierende erste Nukleinsäureamplifikat aus der ersten Reaktion zur Amplifizierung von Nukleinsäuren zu Reaktanden der zweiten Reaktion zur Amplifizierung von Nukleinsäuren abzubauen;
c) Inaktivieren des Abbauenzyms; und
d) Amplifizieren der mit Bisulfit behandelten Nukleinsäurevorlage der zweiten Reaktion zur Amplifizierung von Nukleinsäuren zum Bereitstellen eines zweiten Nukleinsäureamplifikats, dem es an jeglichem kontaminierenden Amplifikat mangelt oder im Wesentlichen mangelt,
(i) wobei es sich bei dem Abbauenzym um ein sequenzspezifisches Restriktionsenzym handelt,
(ii) wobei in jeder Reaktion zur Amplifizierung von Nukleinsäuren mindestens ein Oligonukleotid-Primer verwendet wird, der an die Probenvorlage mit einer oder mehr Fehlpaarungen bindet, aber an jede amplifizierbare kontaminante Nukleinsäure ohne die eine oder mehreren Fehlpaarungen bindet, wodurch ein Restriktionsort in die erste und kontaminierende Nukleinsäure eingeführt wird,
(iii) wobei der gleiche Satz Oligonukleotid-Primer in jeder Amplifizierungsreaktion verwendet wird.

2. Verfahren nach Anspruch 1, wobei das Abbauenzym insbesondere die zweistrangigen Nukeinsäuren abbauen, die zu dem Oligonukleotid-Primer hybridisieren, ohne eine Fehlpaarung zu bilden.

3. Verfahren nach Anspruch 1 oder 2, wobei das Abbauenzym ein thermisch labiles Restriktionsenzym ist und wobei die Sequenz der Nukleinsäurevorlage nicht die Erkennungssequenz des Restriktionsenzyms umfasst.

4. Verfahren nach Anspruch 3, wobei sich das Restriktionsenzym nicht an einer Stelle innerhalb des zu amplifizierenden Fragments spaltet.

5. Verfahren nach Anspruch 3, wobei das Restriktionsenzym Sequenzen erkennt, die nicht in der mit Bisulfit behandelten DNS auftreten.

6. Verfahren nach Anspruch 3, wobei der Erkennungsort des Restriktionsenzyms eine der Sequenzen aus der Gruppe bestehend aus GGCC, AGCT, TGCA, GTAC, und CATG umfasst.

## Revendications

1. Procédé d'élimination de contamination par rémanence lors de réactions d'amplification d'acides nucléiques, dans lequel la matrice est un acide nucléique traité par des bisulfites et comprenant les étapes suivantes :
a) l'amplification d'une première matrice d'acide nucléique traitée par des bisulfites, afin d'obtenir un premier amplificat d'acide nucléique lors d'une première réaction d'amplification d'acides nucléiques ; et
b) avant une deuxième amplification, au cours de laquelle la matrice est un autre acide nucléique traité par des bisulfites, l'ajout d'une enzyme de dégradation appropriée pour dégrader n'importe quel premier amplificat contaminant d'acide nucléique à partir de ladite première réaction d'amplification d'acides nucléiques en réactants de ladite deuxième réaction d'amplification d'acides nucléiques ;
c) l'inactivation de ladite enzyme de dégradation ; et
d) l'amplification de la matrice d'acide nucléique traitée par des bisulfites de la deuxième réaction d'amplification d'acides nucléiques, afin d'obtenir un deuxième amplificat d'acide nucléique auquel manque, ou manque essentiellement, n'importe quel amplificat de contamination,
(i) dans lequel ladite enzyme de dégradation est une enzyme de restriction spécifique de séquence,
(ii) dans lequel, lors de chaque réaction d'amplification d'acides nucléiques, au moins un oligonucléotide d'amorçage sert à la liaison à la matrice échantillon avec au moins un mésappariement, mais se lie à tout acide nucléique contaminant amplifiable sans le ou les mésappariements, ce qui introduit un site de restriction dans ledit premier acide nucléique contaminant,
(iii) dans lequel le même jeu de oligonucléotides d'amorçage est utilisé dans chaque réaction d'amplification.

2. Procédé selon la revendication 1, dans lequel ladite enzyme de dégradation dégrade spécifiquement les acides nucléiques à double brin qui se sont hybridés en oligonucléotide d'amorçage sans former de mésappariement.

3. Procédé selon la revendication 1 ou 2, dans lequel ladite enzyme de dégradation est une enzyme de restriction thermolabile, et dans lequel la séquence de matrice d'acides nucléiques ne comprend pas la séquence de reconnaissance de ladite enzyme de restriction.

4. Procédé selon la revendication 3, dans lequel ladite enzyme de restriction ne se divise pas en une position située dans le fragment à amplifier.

5. Procédé selon la revendication 3, dans lequel ladite enzyme de restriction reconnaît les séquences ne se produisant pas dans l'ADN traité au bisulfite.

6. Procédé selon la revendication 3, dans lequel ledit site de reconnaissance d'enzyme de restriction comprend l'une des séquences provenant du groupe constitué de GGCC, AGCT, TGCA, GTAC et CATG.
